# EUROPEAN PATENT SPECIFICATION

(11) **EP 1 246 913 B1**
(45) Date of publication and mention of the grant of the patent: **05.08.2009**
(21) Application number: 00989415.5
(22) Date of filing: 22.12.2000
(51) Int. Cl.: C12N 15/11, C12N 15/86, C12N 15/63, C12N 5/10, C12N 5/22, C12N 1/21, A61K 39/21

(54) **MOLECULAR CLONES WITH MUTATED HIV GAG/POL, SIV GAG AND SIV ENV GENES**
MOLEKULARE KLONE MIT MUTIERTEN HIV GAG/POL, SIV GAG UND SIV ENV GENE
CLONES MOLECULAIRES AVEC GENES MUTES VIH GAG/POL, VIS GAG ET VIS ENV

(30) Priority: 23.12.1999 US 173036 P
(43) Date of publication of application: 09.10.2002
(73) Proprietor: The Government of the United States of America, as represented by the Secretary, Department of Health and Human Services, Rockville, MD 20852 (US)
(72) Inventor: PAVLAKIS, George, N., Rockville, MD 20850 (US)
(74) Representative: Campbell, Patrick John Henry
(86) International application number: PCT/US2000/034985
(87) International publication number: WO 2001/046408

(56) References cited:
- WO-A-00/39302
- WO-A-00/65076
- US-A- 5 965 726
- US-A- 5 972 596
- QIU J., SONG R. ET AL: "Evaluation of a novel human immunodeficiency virus type 1 Gag DNA vaccines for protein expression in mammalian cells and induction of immune responses." J. VIROL., vol. 73, no. 11, November 1999 (1999-11), pages 9145-9125, XP002172801 cited in the application
- SCHWARTZ S., CAMPBELL M., ET AL.: "Mutational inactivation of an inhibitory sequence in human immunodeficiency virus type 1 results in rev-independent gag expression." J. VIROL., vol. 66, no. 12, December 1992 (1992-12), pages 7176-7182, XP001007821 cited in the application
- SCHNEIDER R ET AL: "Inactivation of the human immunodeficiency virus type 1 inhibitory elements allows rev-independent expression of gag and gag/protease and particle formation" JOURNAL OF VIROLOGY,THE AMERICAN SOCIETY FOR MICROBIOLOGY,US, vol. 71, no. 7, July 1997 (1997-07), pages 4892-4903, XP002137891 ISSN: 0022-538X cited in the application
- WAGNER R ET AL: "REV-INDEPENDENT EXPRESSION OF SYNTHETIC GAG-POL GENES OF HUMAN IMMUNODEFICIENCY VIRUS TYPE 1 AND SIMIAN IMMUNODEFICIENCY VIRUS: IMPLICATIONS FOR THE SAFETY OF LENTIVIRAL VECTORS" HUMAN GENE THERAPY, vol. 11, no. 17, 20 November 2000 (2000-11-20), pages 2403-2413, XP000974656 ISSN: 1043-0342
- KOTSOPOULOU E ET AL: "A Rev-independent human immunodeficiency virus type 1 (HIV-1)-based vector that exploits a codon-optimized HIV-1 gag-pol gene" JOURNAL OF VIROLOGY,THE AMERICAN SOCIETY FOR MICROBIOLOGY,US, vol. 74, no. 10, May 2000 (2000-05), pages 4839-4852, XP002140792 ISSN: 0022-538X
- VINNER L. ET AL.: "Gene gun DNA vaccination with Rev-independent synthetic HIV-1 gp160 envelope gene using mammalian codons" VACCINE, vol. 17, 1999, pages 2166-2175, XP002172802
- NALDINI L ET AL: "IN VIVO GENE DELIVERY AND STABLE TRANSDUCTION OF NONDIVIDING CELLS BY A LENTIVIRAL VECTOR" SCIENCE,US,AMERICAN ASSOCIATION FOR THE ADVANCEMENT OF SCIENCE,, vol. 272, no. 5259, 12 April 1996 (1996-04-12), pages 263-267, XP000583652 ISSN: 0036-8075 cited in the application
- ANDRE S ET AL: "INCREASED IMMUNE RESPONSE ELICITED BY DNA VACCINATION WITH A SYNTHETIC GP120 SEQUENCE WITH OPTIMIZED CODON USAGE" JOURNAL OF VIROLOGY,US,THE AMERICAN SOCIETY FOR MICROBIOLOGY, vol. 72, no. 2, 1 February 1998 (1998-02-01), pages 1497-1503, XP002073767 ISSN: 0022-538X
- ZUFFEREY R ET AL: "MULTIPLY ATTENUATED LENTIVIRAL VECTOR ACHIEVES EFFICIENT GENE DELIVERY IN VIVO" NATURE BIOTECHNOLOGY,US,NATURE PUBLISHING, vol. 15, 1 September 1997 (1997-09-01), pages 871-875, XP002056816 ISSN: 1087-0156 cited in the application

## Description

### I. TECHNICAL FIELD

The invention relates to vector systems and host cells comprising mutated HIV-1 *gag*, HIV-1 *pol* and/or SIV *gag* gene sequences. The invention also relates host cells comprising these vector systems.

### II. BACKGROUND

Until recently, gene therapy protocols have often relied on vectors derived from retroviruses, such as murine leukemia virus (MLV). These vectors are useful because the genes they transduce are integrated into the genome of the target cells, a desirable feature for long-term expression. However, these retroviral vectors can only transduce dividing cells, which limits their use for *in vivo* gene transfer in nonproliferating cells, such as hepatocytes, myofibers, hematopoietic stem cells, and neurons.

Lentiviruses are a type of retrovirus that can infect both dividing and nondividing cells. They have proven extremely efficient at providing long-term gene expression (for up to 6 months) in a variety of nondividing cells (such as, neurons and macrophages) in animal models. See, e.g., Amado et al., Science 285:674-676 (July 1999). It has been proposed that the optimal gene transfer system would include a vector based on HIV, or other lentivirus, that can integrate into the genome of nonproliferating cells. Because retroviruses integrate in the genome of the target cells, repeated transduction is unnecessary. Therefore, in contrast to an adenoviral vector capable of in vivo gene delivery, problems linked to the humoral response to injected viral antigens can be avoided. See, e.g., Naldini et al., Science, 272:263-267 (1996), p. 263.

HIV and other lentiviruses have a complex genome that, in addition to the essential structural genes *(env, gag,* and *pol),* contains regulatory (*tat* and *rev*) and accessory genes (*vpr*, *vif, vpu,* and *nef*). HIV has evolved to efficiently infect and express its genes in human cells, and is able to infect nondividing cells such as macrophages because its preintegration complex can traverse the intact membrane of the nucleus in the target cell. This complex contains, in addition to the viral DNA, the enzyme integrase, the product of the *vpr* gene, and a protein encoded by the *gag* gene called matrix. The matrix protein enables the preintegration complex to pass into the nucleus to access the host DNA. Lentiviruses cannot efficiently transduce truly quiescent cells (cells in the Go state). However, unlike murine retroviral vectors, in addition to being able to infect dividing cells, HIV-based vectors can achieve effective and sustained transduction and expression of therapeutic genes in nondividing cells, such as hematopoietic stem cells and in terminally differentiated cells such as neurons, retinal photoreceptors, muscle, and liver cells. See, e.g., Amado et al. (July 1999) and Klimatcheva et al., Frontiers in Bioscience 4:d481-496 (June 1999), and the references cited therein.

Although lentiviral vectors can be efficient gene delivery vehicles, there are safety concerns due to their origin. Therefore, the field has turned its attention to the development of vectors and production systems with built-in safety features to prevent the emergence of replication competent lentivirus (RCL). For example, in most laboratory applications, lentiviral vectors are generally created in a transient system in which a cell line is transfected with three separate constructs: a packaging construct, a transfer construct, and an envelope encoding construct. The packaging construct contains the elements necessary for vector packaging (except for env) and the enzymes required to generate vector particles. The transfer construct contains genetic cis-acting sequences necessary for the vector to infect the target cell and for transfer of the therapeutic (or reporter) gene. The lentivirus *env* gene is generally deleted from the packaging construct and instead the envelope gene of a different virus is supplied in a third vector "the env-coding vector", although the lentiviruses env gene may be used if it is desired that the vector be intended to infect CD4⁺T cells. A commonly used envelope gene is that encoding the G glycoprotein of the vesicular stomatitis virus (VSV-G), which can infect a wide variey of cells and in addition confers stability to the particle and permits the vector to be concentrated to high titers (see, e.g., Naldini et al., Science 272:263-267 (1996) and Akkina et al. J. Virol. 70:2581 (1996). The use of three separate constructs and the absence of overlapping sequences between them minimizes the possibility of recombination during lentivirus (transfer) vector production. In addition, because no viral proteins are expressed by the lentiviral (transfer) vector itself, they do not trigger an effective immune response against cells expressing vector in animal models (a particular problem with vectors based on adenovirus). See, e.g., Amado et al., Science 285:674-676 (July 1999) and the references cited therein. See also Naldini et al. Science 272:263-267 (1996).

The initial packaging plasmids contained most HIV genes except for *env*. In an effort to improve safety, subsequent HIV vectors have been produced in which the packaging plasmid is devoid of all accessory genes. This process does not interfere with efficient vector production and significantly increases the safety of the system because potential RCLs lack the accessory genes necessary for efficient replication of HIV in humans. Although these vectors can transduce growth-arrested cell lines and neurons *in vivo,* they have been reported to not efficiently transduce macrophages. The accessory gene *vpr* is believed to be necessary for HIV infection of these cells using these HIV vectors. See, Zufferey et al., Nature Biotechnol. 15:871-875 (1997). In contrast, as discussed later herein, the HIV-based lentiviral vectors of the present invention do not need any HIV accessory genes in order to be able to infect human macrophages and the other cells tested.

The requirement of *vpr* or *vif* for efficient transduction of liver cells has also been reported. See, e.g., Kafri et al., Nature Genet. 17:314 (1997). These results indicate that the requirement of accessory genes for efficient lentivirus-mediated gene transfer is dependent on the type of cell chosen as target, suggesting that future applications of lentiviral vectors may involve vector constructs with different accessory genes, as needed.

Zufferey et al., (1997) describe an HIV vector system in which the virulence genes, *env, vif, vpr, vpu,* and *nef* have been deleted. This multiply attenuated vector conserved the ability to transduce growth-arrested cells and monocyte-derived macrophages in culture, and could efficiently deliver genes in vivo into adult neurons. The packaging plasmids described Zufferey et al. (1997) and Naldini et al. (1996) encode Rev and Tat, in addition to Gag and Pol.

Lentiviral vectors engineered to become packaged into virions in the absence of the regulatory gene *tat* have also been described. See, e.g., Kim et al., J. Virol. 72:811-816 (1998) and Miyoshi et al. J. Virol. 72:8150-8157 (1998). In these vectors the *tat* gene has been removed from the packaging plasmid. Kim et al. state that *tat* is not necessary as long as the serial 5' LTR promoter is replaced with a strong constitutive promoter. It also has other advantages for HIV therapy. Replacement of the HIV-1 LTR with a constitutive HCMV promoter permits the use of anti-Tat molecules such as Tat transdominant mutants or Tat activation response element decoys as therapeutic agents, since they will not affect vector production. (see p. 814, col. 2). The removal of the *tat* gene eliminates an essential virulence factor that could contribute to a possible RCL. Kim et al. (1998) describe a vector system which does not contain *tat, vif, vpr, vpu* and *nef.* The preferred vector system includes the rev gene which, the authors state "with RRE, is required for efficient RNA handling in this system." (p. 811, col. 2). However, Kim et al. also constructed Rev independent constructs using CTE. Kim et al. state that the *rev*/RRE components could be removed by using a sequence such as the Mason-Pfizer monkey virus (MPMV) constitutive transport element (CTE), thereby eliminating all accessory proteins, but this leads to a significant reduction in titer.

Srinivasakumar et al., J. Virol. 71:5841-5848 (1997) describes the generation of stable HIV-1 packaging lines that constitutively express high levels of HIV-1 structural proteins in either a Rev-dependent or a Rev-independent fashion. These cell lines were used to assess gene transfer by using a HIV-1 vector expressing the hygromycin B resistance gene and to study the effects of Rev, Tat, and Nef on the vector titer. The Rev-independent cell lines were created by using *gag-pol* and *env* expression vectors that contain the MPMV CTE. This article describes the construction of four plasmids, among others: CMV gagpol-RRE and pCMVenv, which require Rev coexpression for HIV-1 structural gene expression, and pCMV gagpol-CTE and pCMVenv-CTE, which do not. To create Rev-containing and Rev-independent packaging, cell lines, CMT3 cells were transfected with vectors expressing Gag, Gag-Pol, and Env, using a calcium phosphate transfection procedure.

By creating an HIV vector which contained the MPMV CTE (pTR167-CTE) and a packaging cell line which expressed the HIV structural proteins in a Rev-independent fashion, the authors were able to obtain a HIV vector system that functions completely without Rev. The titer of the vector obtained from this system was essentially the same as that obtained from a parallel system which contained Rev. The authors state that, in this context, the CTE seemed to substitute completely for Rev-RRE functions, similar to what was previously observed in transient-expression assays with Rev-dependent constructs. This is in contrast to situations where several rounds of HIV replication were measured. In those cases, titers from CTE-containing viruses were always reduced by at least 1 log unit compared to viruses utilizing Rev and the RRE. (See, Srinivasakumar et al., p. 5847).

The authors state that the advantages of having a HIV vector system that works in the absence of Rev opens the possibility of using it as a delivery vehicle for intracellular immunization against Rev function. Genes encoding Rev antagonists that have dramatic inhibitory effects on HIV replication, such as Rev M10 or RRE decoys, could be introduced into an HIV vector and put into cells normally infectable by HIV. Expression of the "anti-Rev" gene would be expected to dampen HIV infection. Any residual HIV replication should lead to activation of the vector LTR (by Tat) and create a vector-derived RNA that would be packaged by proteins derived from the infectious virus. In this scenario, the wild-type virus would act as a helper that may allow the spread of vector particles to previously nonimmunized cells. Because of the additional vector spread, it is likely that this type of scheme will be more effective in modulating HIV infection *in vivo* than one based on traditional retrovirus vectors. The authors state that they are currently testing this approach in model systems. (See, Srinivasakumar et al., p. 5847).

Another development in the quest for a safe system is the so-called self-inactivating (SIN) vector. See, e.g., Yu et al., Proc Natl Acad Sci USA 83:3194-8 (1986) and Miyoshi et al., J. Virol. 72:8150 (1998). In Yu et al., a retrovirus-derived vector SIN vector was designed for the transduction of whole genes into mammalian cells. The SIN vector of Yu et al. contains a deletion of 299 base pairs in the 3' long terminal repeat (LTR), which includes sequences encoding the enhancer and promoter functions. When viruses derived from such vectors were used to infect NIH 3T3 cells, the deletion was transferred to the 5' LTR, resulting in the transcriptional inactivation of the provirus in the infected cell. Introduction of a hybrid gene (human metallothionein-promoted c-fos) into cells via a SIN vector was not associated with rearrangements and led to the formation of an authentic mRNA transcript, which in some cases was induced by cadmium. The vector described in Miyoshi et al. also contains a deletion the 3' (downstream) LTR. A sequence within the upstream LTR serves as a promoter under which the viral genome is expressed. The deletion introduced in the downstream LTR is transferred to the upstream LTR during reverse transcription. This deletion inactivates the LTR promoter and eliminates the production of vector RNA. The gene (or genes) to be transferred (e.g., a reporter or therapeutic gene) is expressed from an exogenous viral or cellular promoter that is inserted into the lentivirus vector. An important safety feature of SIN vectors is that inactivation of the promoter activity of the LTR reduces the possibility of insertional mutagenesis (of the transfer vector) into the host genome. In addition, because the expression of the (transfer) vector RNA is eliminated, the potential for RCL production in the target cell is further minimized. SIN vectors should be particularly useful in gene transfer experiments designed to study the regulated expression of genes in mammalian cells. Absence of enhancer and promoter sequences in both LTRs of the integrated provirus should also minimize the possibility of activating cellular oncogenes and may provide a safer alternative to be used in human gene therapy. Other modifications to enhance safety and specificity include the use of specific internal promoters that regulate gene expression, either temporally or with tissue or cell specificity.

Other strategies to improve safety in human studies would be to use nonhuman lentiviruses such as simian immunodeficiency virus, bovine immunodeficiency virus, or equine infectious anemia virus. Of these, vectors derived from the feline immunodeficiency virus have been engineered to efficiently transduce nondividing human cells. See, e.g., Poeschla et al., Nature Med. 4:354-357 (1998) and WO 99/15641. In addition, White et al., J. Virol. 73:2832-2840 (April 1999) described lentiviral vectors using human and simian immunodeficient virus elements in attempt to improve safety by reducing the likelihood of recombination between packaging constructs and transfer constructs.

The development of efficient packaging lines has proven challenging because expression of the VSV-G envelope and a number of HIV proteins is toxic to cells. Recently, a producer line has been designed in which the expression of packaging genes and VSV-G, and therefore the production of vector, can be turned on at will. Kafri et al., J. Virol. 73-576-584 (1999). The cell line can be expanded for scale-up vector production when the expression of toxic genes is turned off. This cell line produces high titer vector without generating RCL. Hematopoietic stem cells transduced with an HIV vector were transplanted into rhesus macaques as described by Donahue et al. Blood 92 (suppl. 1), abstract 4648.5 (1998) with at least a 14-month follow-up. At that time the procedure proved to be safe; all animals in the study have remained healthy without evidence of circulating HIV or vector. See, Amado et al., Science 285:674-676 (July 1999).

Many gene therapy protocols have been designed to correct a number of inherited metabolic, infectious, or malignant diseases using the hematopoietic stem cell. This cell has the capacity to self-renew and to differentiate into all of the mature cells of the blood and immune systems. Many diseases that affect these systems could potentially be treated by the stable introduction of therapeutic genes into stem cells. Recently, lentiviral vectors were shown to bypass the need for *ex vivo* stem cell stimulation (which is necessary when using murine retroviral vectors), by mediating efficient gene transfer into very primitive human stem cells that contributed to stable, long-term reconstitution of SCID mouse bone marrow with many hematopoietic lineages. See, e.g., Miyoshi et al., Science 283:682 (1999). Similarly, in a rhesus macaque model of autologous transplantation with lentivirus-transduced stem cells, multilineage gene expression was found, suggesting transduction of an early blood cell progenitor under conditions of minimal stem cell stimulation, ordinarily insufficient for transduction with murine retroviruses. See, Donahue et al., Blood 92 (suppl. 1), abstract 4648.5 (1999) and Amado et al., Science 285:674-676 (July 1999).

In HIV infection, another advantage of lentiviral vectors designed against HIV is their potential to be mobilized by HIV in the infected patient, because the virus supplies all of the necessary elements for packaging of the vector. If these mobilized vectors contained the HIV envelope, they could efficiently transfer their genes (for example, genes custom-designed to confer resistance against HIV) into CD4⁺ T cells, protecting them from subsequent HIV infection. Lentiviral vectors can also be designed to efficiently express their genes only in CD4⁺ T cells that are infected with HIV (so called tat-inducible vectors). In these vectors, all HIV genes, including *tat* and *rev,* are ablated; cis-acting sequences required for integration, expression, and packaging are retained, and expression is dependent on the activity of the HIV LTR (which requires transactivation by Tat). It has been shown that in this system, vector expression is induced efficiently upon HIV infection. Moreover, in the absence of genes that confer resistance against HIV, stable integration of this vector in permissive cell lines resulted in inhibition of HIV replication. Although the mechanism of HIV inhibition has not been completely elucidated, preliminary results suggest that this vector competes with HIV at the level of reverse transcription. See, An et al., J. Virol., in press, and Amado et al., Science 285:674-676 (1999).

A number of other potential medical applications, where the modification of the genetic material of quiescent cells could result in the prevention or reversal of a disease process, are beginning to be explored. For example, the finding that lentiviral vectors can mediate stable and long-term gene transfer by direct injection of vector into the rat and mouse retina has lent support to the notion of gene therapy for the treatment of retinitis pigmentosa. This degenerative disease of the retina is characterized by photoreceptor cell death, resulting in a slow progression to blindness. Mutations in the cGMP phosphodiesterase β subunit (PDEβ) gene of rod photoreceptors lead to an autosomal recessive form of retinitis pigmentosa in humans, and in the *rd* mouse model of the disease. Previous studies have shown that adenovirus and adeno-associated virus-mediated PDEP subretinal gene transfer results in a delay in photoreceptor cell death. Using the *rd* mouse model, a recent study demonstrated that photoreceptors could be rescued in up to 50% of eyes injected with a lentivirus vector containing the murine PDEβ gene. In contrast with the short-term expression previously obtained with adenovirus vectors, PDEβ expression in this study persisted for at least 24 weeks. This finding points to the potential success of gene therapy in a disease that currently lacks effective treatment. See, Takahashi et al., J. Virol., 73:7812-7816 (Sept. 1999) and Amado et al. Science, 285:674-676 (1999).

In nature, the expression of *gag, pol,* and env of HIV-1 depends on the presence of the viral Rev protein. This dependence is, at least in part, due to the presence of negatively acting sequences (inhibitory or instability elements [INS]) located within unspliced and partially spliced mRNAs. The positive interaction of Rev with the Rev-responsive element [RRE] in these mRNAs counteracts the negative effects of the inhibitory sequences.

None of the above references teach or suggest that the *gag* and/or *pol* genes described therein may be replaced with the *gag* and/or *pol* genes in which the inhibitory/instability have been mutated to render their expression Rev-idependent. Furthermore, there is no disclosure of the specific HIV-1 *gaglpol* or SIV *gag* mutated genes described herein.

The *gaglpol* clone of the invention was made using the method for eliminating inhibitory/instability regions from a gene as first described in U.S. patent application Serial No. 07/858,747, filed March 27, 1992, (inventors, G. Pavlakis and B. Felber) entitled "Method of Eliminating Inhibitory/Instability Regions from mRNA" and later described in a Continuation-in-Part ("CIP") application, filed as PCT application PCT/US93/02908 on March 29, 1993 and U.S. Patent Nos. 5,972,596 and 5,965,726. The disclosure of the CIP application was published as International Publication No. WO 93/20212 on October 14, 1993.

The method was also described in Schwartz et al., J. Virol. 66:7176-7182 (1992).

Schneider et al., J. Virol. 71:4892-4903 (1997), extend the work described in the patent applications and in Schwartz et al. by identifying and characterizing additional INS within *gag, protease* and *pol* genes and mutating them in a similar manner. Schneider et al. disclose nucleic acid constructs which contain completely mutated HIV-1 *gag* genes, but only partially mutated HIV-1 *pol* genes.

Schneider et al. demonstrate that expression vectors containing an intact or nearly intact p55*^{gag}* region allow the production of immature viral particles in mammalian cells in the absence of any other HIV proteins. The introduction of additional mutations in the *protease* region allowed efficient production of Gag/protease, which resulted in processing of the Pr55*^{gag}* precursor and production of mature Gag particles with a lentivirus-like conical-core structure.

Schneider et al. disclose that Rev-independent expression vectors allow the efficient expression of Gag proteins in many cell lines that are not able to support efficient Rev-RRE-dependent rescue of these RNAs. Schneider et al. also disclose that *gag*/*pol* expression vectors may be important for vaccination approaches against HIV-1, since the *gag*/*pol* region is more conserved than is the *env* region and may be important for an effective immune response against HIV and for protection against infection. They also state that efficient HIV gene expression in many cells is also of interest for possible gene transfer experiments using lentiviral vectors in nondividing or slowly dividing cells, since HIV and the other lentiviruses are able to infect quiescent cells.

Pavlakis et al., Natl Conf Hum Retroviruses Relat Infect (2nd). (1995), 91, state that Rev-independent Gag expression vectors were able to produce viral particles in human and mouse cells in the absence of any other HIV proteins, and that additional mutations in the *pol* region allowed the expression of the protease and the processing of the p55 gag precursor. Direct DNA injection of TAT and Rev independent Gag expression vectors in mouse muscle resulted in Gag expression detected by ELISA and in anti-gag antibody response. Several Rev-and Tat- independent Gag expression cassettes were inserted into retroviral vectors and cell lines expressing Gag or Gag fragments that are dominant negative inhibitors of HIV-1 were constructed.

Shiver et al. (1996) describe the results of DNA vaccination of mice and non-human primates with mutated plasmid DNA encoding either mutated genes encoding HIV-1 gag (p55 gag) or env (gp120 or gp160). Both *gag* and *env* vaccine recipients exhibited antigen-specific cytotoxic and helper T lymphocyte (CTL, Th) responses. The results are stated to demonstrate the DNA vaccines elicited long-lived T cell responses in both mice and nonhuman primates that were disseminated throughout the lymphatics.

### III. SUMMARY OF THE INVENTION

The invention provides a lentiviral expression system which is capable of functioning in the absence of Rev, Tat, and any viral RNA transport element comprising the following:
(a) a packaging vector comprising a HIV-1 *gag*/*pol* gene which has been mutated to eliminate inhibitory/instability regions;
(b) a transfer vector; and
(c) an envelope encoding vector.

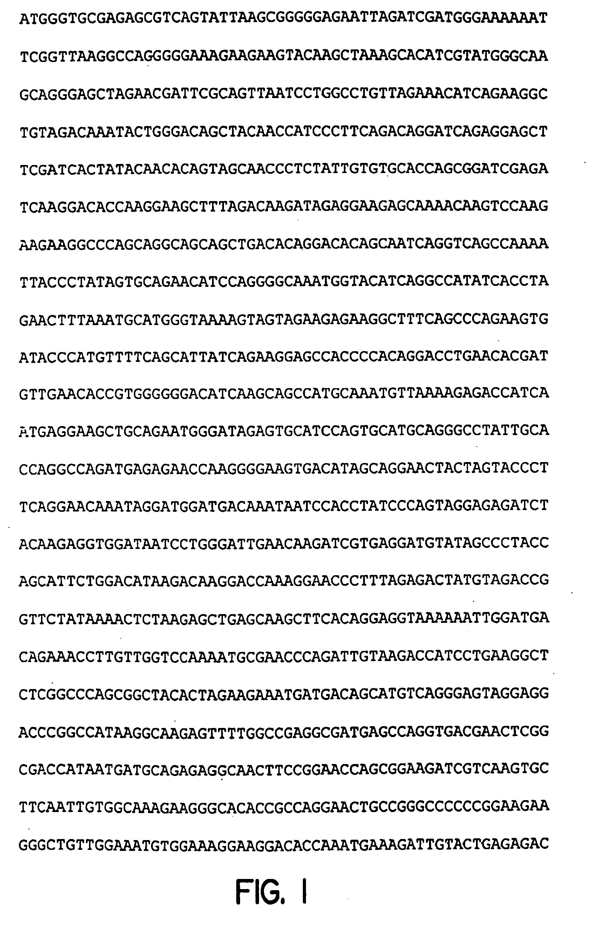

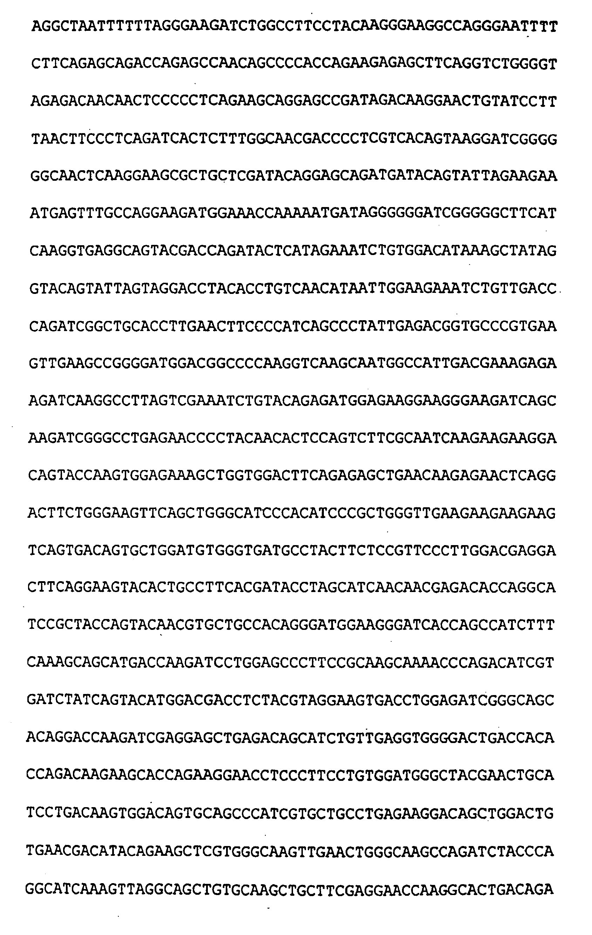

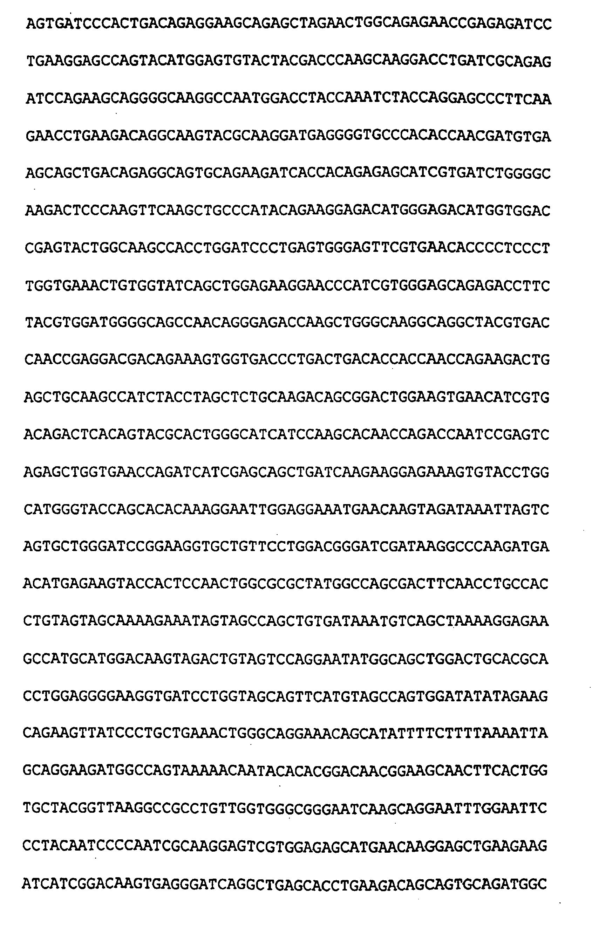

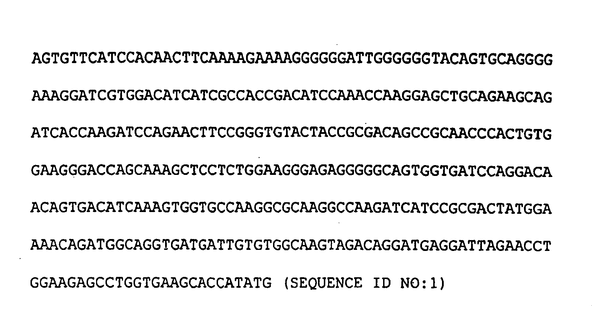

The mutated HIV-1 *gag*/*pol* gene may be that shown in Figure 1 (SEQUENCE ID NO:1).

The invention also relates to host cells comprising the vector system.

The invention also provides a process for making a lentiviral particle in the absence of Rev, Tat, or any viral RNA transport element comprising expressing HIV Gag and HIV Pol in a host cell from a HIV-1 *gag*/*pol* gene which has been mutated to eliminate inhibitory/instability regions and expressing an envelope protein from an envelope encoding gene whose expression is independent of Rev, Tat, or any viral RNA transport element.

Furthermore, the invention provides a process for making a lentiviral particle comprising expressing HIV Gag and HIV Pol in a host cell from a vector comprising the nucleotide sequences encoding HIV Gag and HIV Pol corresponding to Seq. ID No. 1 in the presence of a gene encoding an envelope protein.

The vector systems and host cells may be used for immunotherapy and immunoprophylaxis, e.g., as a vaccine, or in genetic therapy after expression, preferably in humans. The nucleic acid constructs incorporated into lentiviral vectors may be directly injected into tissue cells resulting in efficient expression of the encoded protein or protein fragment.

### IV. BRIEF DESCRIPTION OF THE DRAWINGS

Fig. 1. DNA sequence of a mutated HIV-1 *gag*/*pol* molecular clone (SEQUENCE ID NO:1). The gagpol terminator is located at positions 4305-4397 of SEQUENCE ID NO:1.
Fig. 2. Comparison of the sequence of the wild -type and mutated *pol* region in pCMV gagpolBNkan*.* Position #1 in the figure is position 2641 in plasmid pCMVgagpolBNkan. The comparison starts at position 1872 from the gag initiator ATG.
Fig. 3. DNA sequence of a mutated SIV *gag* molecular clone (SlVgagDX).
Fig. 4. Comparison of the mutated SIV gag DNA sequence in SlVgagDX with the wild type SIV sequence from Simian (macaque) immunodeficiency virus isolate 239, clone lambda siv 239-1 (GenBank accession No. M33262).
Fig. 5. Schematic diagram of some components of sample versions of a lentiviral system. BGH poly (A): bovine growth hormone poly (A) signal; MSD: mutated splice donor site; ψ: encapsidation signal; SD, splice donor site; SA, splice acceptor site; "X" indicates that the ATG codon of the partial *gag* gene sequence is mutated so that translation of this gene does not occur.
Fig. 6. Schematic diagram of the packaging construct pCMVgagpolBNkan.
Fig. 7. Schematic diagram of transfer construct 1: pmBCwCNluci. The packaging signal, the CMV promoter and the coding region for the luciferase gene are flanked by the 5' and 3 HIV-1 LTRs, which provide promoter and polyadenylation signals, as indicated by the arrows. Three consecutive arrows indicate the U5, R, and U3 regions of the LTR, respectively. The transcribed portions of the LTRs are shown in black. Some restriction endonuclease cleavage sites are also indicated.
Fig. 8. Schematic diagram of transfer construct 1: pmBCmCNluci. Symbols are as above.

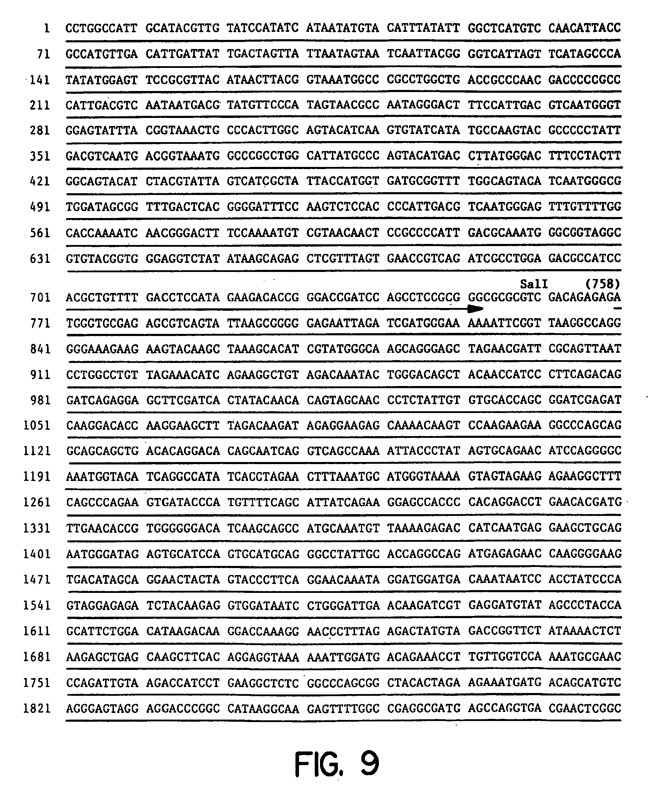

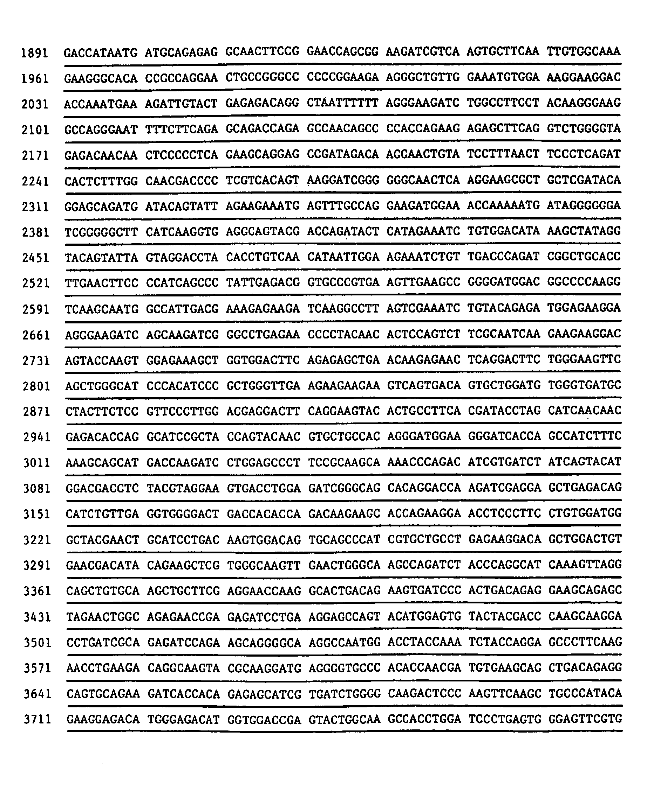

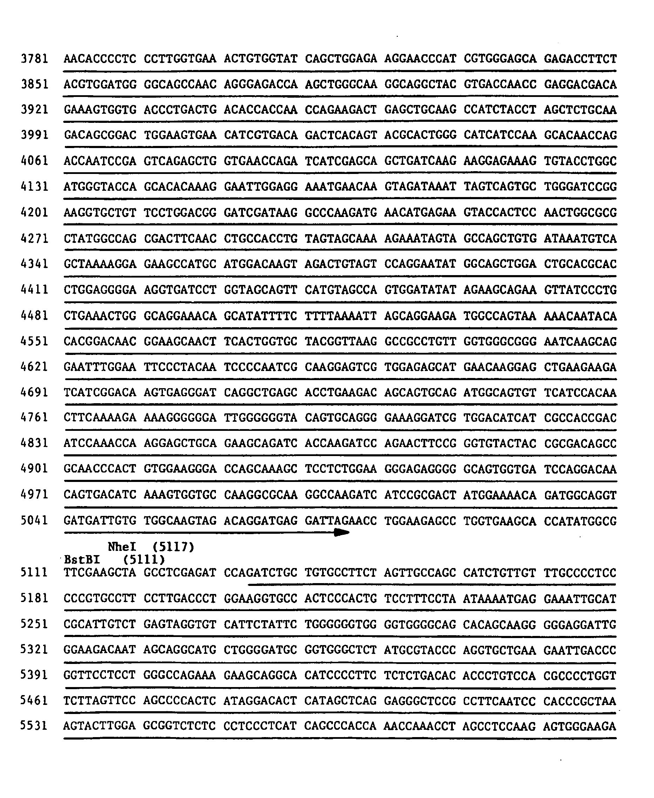

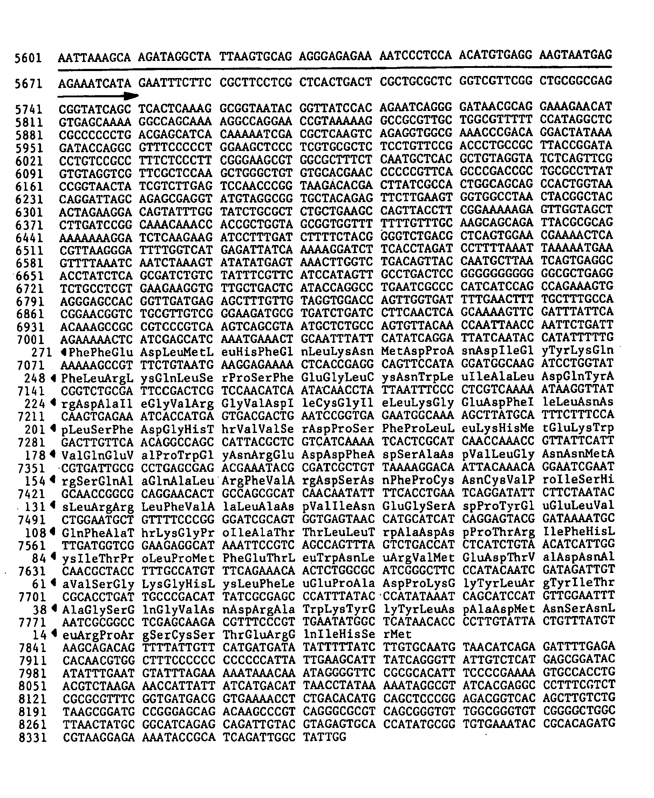

Fig. 9. DNA sequence of packaging construct pCMVgagpolBNkan.
Fig. 10. DNA sequence of transfer construct 1: pmBCwCNluci.
Fig. 11. DNA sequence of transfer construct 1: pmBCmCNluci. Figure 12:
Fig. 12. Nucleotide sequence of the region BssHII (711) to ClaI (830) in wild-type HIV-1 molecular clones HXB2 and NL4-3, and in the transfer constructs. The translation initiator signal for Gag protein (ATG) is underlined. pmBCwCNluci and pmBCmCNluci (transfer constructs 1 and 2) contain the sequence mBCwCN. Transfer construct 3 contains the sequence m2BCwCN. In contrast to the sequence mBCwCN, m2BCwCN has different mutations at the 5' splice site region and has an intact Gag ATG.
Fig. 13. Bar graph showing levels of gag protein that is released from cells upon transient transfection with pCMVgagpolBNkan (labeled pCMVBNKan in the figure).
Fig. 14. Bar graph showing reverse transcriptase activity from the Rev-independent gag-pol HIV-1 vector pCMVgagpolBNkan (labeled pCMVBNKan in the figure).
Fig. 15. Bar graphs showing the amount of luciferase per nanogram of p24 Gag protein detected in cells transducted with PCMVgagpolBNkan Rev-independent *gag*-HIV-1 based retroviral vectors. The results show that with PCMVgagpolBNkan Rev-independent gag-HIV-1 based retroviral vectors display high transduction efficiency in (A) 293 cells, (B) human lymphoid cells, (C) human myeloid cells (U937), as well as (D) non-dividing cells such as primary human macrophages.
Fig. 16. Schematic diagram of the SIV envelope encoding vector CMVkan/R-R-SIVenvCTE.
Fig. 17. DNA sequence of the SIV envelope encoding vector CMVkan/R-R-SIVenvCTE containing a mutated SIV env gene.

### V. MODES FOR CARRYING OUT THE INVENTION

It is to be understood that both the foregoing general description and the following detailed description are exemplary and explanatory only, and are not restrictive of the invention, as claimed. The accompanying drawings, which are incorporated in and constitute a part of the specification, illustrate an embodiment of the invention and, together with the description, serve to explain the principles of the invention.

One aspect of the disclosure comprises vectors that encode the Gag and/or Pol of HIV-1 in a Rev-independent manner. An example of such a vector which is described herein is the plasmid pCMVgagpolBNkan, which encodes the complete Gag and Pol of HIV-1 in a Rev-independent manner, and also contains a gene conferring kanamycin resistance. This plasmid is Tat and Rev-independent and was generated by eliminating the inhibitory/instability sequences present in the *gag*/*pol* mRNA without altering the amino acid sequence of the proteins coded by the genes.

The *gag*/*pol* clone of the disclosure is a DNA construct of the *gag*/*pol* region of HIV which has had the inhibitory/instability regions removed. The construct is expected to be useful as a component a new type of lentivirus vector for use in gene therapy or as a vaccine.

The *gag, pol* or *gag*/*pol* sequences can be highly expressed in human and other mammalian cells in the absence of any other regulatory and structural protein of HIV, including Rev. When the *gag*/*pol* sequences are combined with a sequence encoding an envelope protein, such as the VSV G protein or the HIV envelope protein (e.g., in the same vector or in another expression vector), infectious virus is produced after transfection into human cells. When a gene encoding a non-HIV envelope protein is used, for example, in the presence of the HIV *gaglpol* gene, the virus particles produced would contains only the HIV proteins Gag and Pol.

Lentiviral vectors or vector systems based on the *gag, pol* or *gag*/*pol* sequences of this disclosure, as exemplified by the Rev-independent pCMVgagpol BNkan construct described herein, may be used for gene therapy *in vivo* (e.g., parenteral inoculation of high titer vector) or *ex vivo* (e.g., *in vitro* transduction of patient's cells followed by reinfusion into the patient of the transduced cells). These procedures are been already used in different approved gene therapy protocols.

The HIV *gag*/*pol* clone and SIV *gag* clone of the disclosure made using the method for eliminating inhibitory/instability regions from a gene as described in U.S. patent application Serial No. 07/858,747, filed March 27, 1992, and also in related U.S. Patent Nos. 5,972,596 and 5,965,726

This method does not require the identification of the exact location or knowledge of the mechanism of function of the INS. Generally, the mutations are such that the amino acid sequence encoded by the mRNA, is unchanged, although conservative and non-conservative amino acid substitutions are also envisioned where the protein encoded by the mutated gene is substantially similar to the protein encoded by the non-mutated gene. The mutated genes can be synthetic (e.g., synthesized by chemical synthesis), semi-synthetic (e.g., a combination of genomic DNA, cDNA, or PCR amplified DNA and synthetic DNA), or recombinantly produced. The genes also may optionally not contain introns. The nucleic acids of the invention may also contain Rev-independent fragments of these genes which retain the desired function (e.g., for antigenicity of Gag or Pol, particle formation (Gag) or enzymatic activity (Pol)), or they may also contain Rev-independent variants which have been mutated so that the encoded protein loses a function that is unwanted in certain circumstances. In the latter case, for example, the gene may be modified to encode mutations (at the amino acid level) in the active site of reverse transcriptase or integrase proteins to prevent reverse transcription or integration. Rev-independent fragments of the gag gene are described in U.S. patent application Serial No. 07/858,747, filed March 27, 1992, and also in related U.S. Patent Nos. 5,972,596 and 5,965,726.

In addition to being capable of producing HIV Gag and Pol proteins in the absence of Rev regulatory protein in a cell in vivo, the HIV *gaglpol* clone and SIV *gag* clone of the disclosure are also capable of producing HIV Gag and Pol proteins in the absence of any added cis acting transport element, such as CTE or CTE-like elements (collectively refered herein as RNA Transport Elements (RTE)). Experiments indicate that the mutated vectors of the disclosure for SIV gag are far superior to those adding CTE (see Qiu et al., J Virol. 73:9145-52 (1999)).

The expression of the proteins encoded by these vectors after transfection into human cells may be monitored at both the level of RNA and protein production. RNA levels are quantitated by methods known in the art, e.g., Northern blots, S1 mapging or PCR methods. Protein levels may also be quantitated by methods known in the art, e.g., western blot or ELISA or fluorescent detection methods. A fast non-radioactive ELISA protocol can be used to detect gag protein (DUPONT or COULTER gag antigen capture assay).

At least three types of lentiviral vectors based on the *gag*/*pol* genes of the disclosure for use in gene therapy and/or as a vaccine are envisioned, i.e., lentiviral vectors having
a) no round of replication (i.e., a zero replication system)
b) one round of replication
c) a fully replicating system

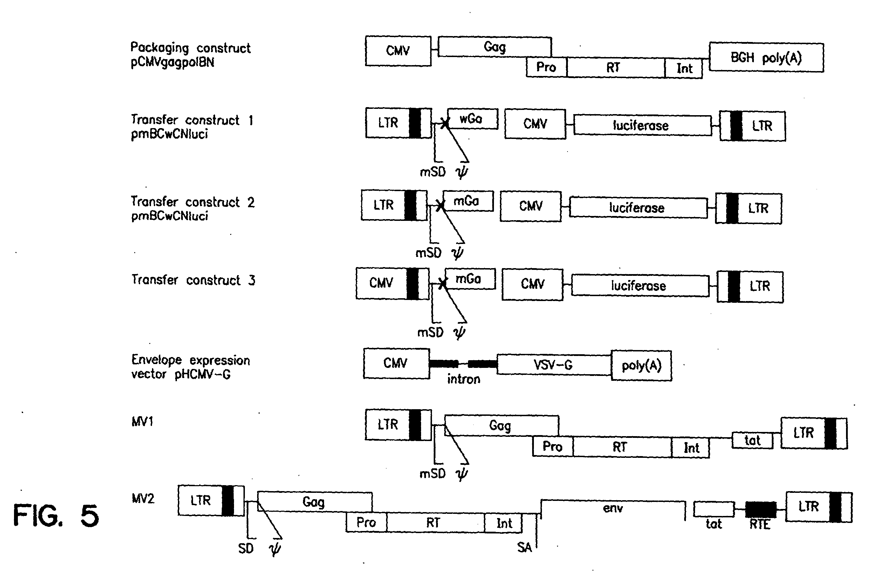

For a system with no round of replication, a *gag*/*pol* gene, or separate *gag* and *pol* genes, or fragments of these genes, expressed using appropriate transcription units, e.g., a CMV promoter and a BGH poly (A) site. This will allow expression of the *gag*/*pol* unit (or *gag* or *pol* or fragment(s) thereof) for vaccine purposes. This expression can be accomplished without the production of any functional retroviral enzymes, provided that the appropriate mutation(s), e.g., a missense mutation, are introduced. In a zero replication system, a virus stock will be administered to the cells or animals of interest. For example, if one creates and uses a virus stock with the exemplified system using the packaging vector PCMVgagpolBNKan, the transfer construct pmBCwCNluci or pmBCmCNluci, and the envelope containing vector pHCMV-G, one obtains a zero replication system. The virus particles produced by such system can infect cells, and the reverse transcribed transfer construct DNA will go into the nucleus but, because the coding regions for viral structural proteins are not present, there will be no virus expression and replication (0 rounds). If one transfects cells *in vivo* with the same 3 DNAs, they will go to the nucleus, express viral proteins, make infectious virus particles and go out and infect another cell or cells (1 round). Since in vivo delivery of three plasmids may result in lower expression, at least two different embodiments are envisioned. In the first, two plasmids may be used, e.g., MV1 shown in Fig. 5 and an envelope expression plasmid such as pHCMV-G. Other plasmids encoding functional envelopes from HIV, SIV, or other retroviruses can also be used. Transfection by the two plasmids results in infectious virus that can infect and integrate into new cells (1 round). The infected cells produce gagpol but virus propagation is not possible in the absence of env.

For a system with one round of replication, at least two additional embodiments of the disclosure are envisioned. In the first method, a combination of the genes, e.g., a *gag*/*pol* gene, an *env* encoding gene and, preferably, a gene encoding a reporter protein or other polynucleotide or protein of interest, are delivered into the cells of interest *in vivo.* As discussed above for the exemplified system, if one transfects cells *in vivo* with the same 3 DNAs, they will go to the nucleus, express viral proteins, make infectious virus particles, be released and infect another cell or cells (1 round).

In another embodiment of the disclosure, the same result (i.e., only one round of replication) can be obtained by using transfer vectors that have deletions in the 3' LTR and in which a heterologous-promoter (e.g., the CMV-promoter, or inducible promoter, or tissue-specific promoter), is used in place of the 3'LTR promoter. The mutations in the 3' LTR making it inactive upon reverse transcription and integration. This is because the integrated provirus derives both its 5' LTR and its 3' LTR from the 3' LTR of the starting (transfer) construct. (This is a well-known property of all retroviruses and has been used to make self-inactivating vectors (SIN)). There are several reasons one may want to inactivate the incoming LTR promoter, one of which is to use a different tissue specific or regulated promoter for expression of a gene of interest in the integrated provirus. Note that, with SIN vectors, if one uses a viral stock made *in vitro* after transfection into cells and collection of infectious virus, there will be no round of replication. If one transfects cells with the DNAs *in vivo,* there will be one round of replication. If functional *gag, pol,* or *env* are not included in the DNA mix, there will not be any infection at all (i.e., infectious viruses will not be made).

A fully replicating Rev-independent system has not been constructed yet, although it is expected that a functional system can be constructed using Rev-independent *gag*/*pol* and *env* sequences. If desired, extra posttranscriptional controls elements such as the CTE element, which can replace Rev and give infectious virus (see e.g., Zolotukhin et al., J.Virol.68:944-7952 (1994)) are included. The fully replicating system should be in one piece, containing the LTR, packaging signal, gag/pol, splice site, env, tat, one or more CTE or CTE-like elements (if desired for optimal results), and LTR. Tat is thought to be required in this construct, at least in non-permissive cells. Such a system is depicted in Figure 5, (construct MV2). In this system, a cell or animal of interest (preferably human) would be infected with virus stock that then propagates. CTE or CTE-like elements (depicted in construct MV2 as RTE (RNA Transport Elements)) are desirable since they have been shown to improve expression, and since many retroviruses require the presence of posttranscriptional control elements. There are several types of CTE and CTE-like elements, and these elements appear to work via a different pathway from the Rev-RRE pathway. See, e.g., Tabernero et al., J Virol. 71:95-101 (1997). See also, Pavlakis and Nappi, PCT/US99/11082, filed May 22, 1999, published as WO 99/61596 on December 2, 1999 (and incorporated herein by reference), which describes a new type of post-transcriptional control element that is able to replace CTE and HIV RRE/Rev. The Pavlakis-Nappi element does not work in the same way as CTE and does not have any sequence or structure homology.

In a preferred embodiment, a lentiviral system of the invention comprises the following three components:
1. a packaging vector containing nucleic acid sequences encoding the elements necessary for vector packaging such as structural proteins (except for HIV *env)* and the enzymes required to generate vector particles, the packaging vector comprising at least a HIV *gag*/*pol* gene which has been mutated to eliminate inhibitory/instability regions;
2. a transfer vector containing genetic cis-acting sequences necessary for the vector to infect the target cell and for transfer of the therapeutic or reporter or other gene(s) of interest, the transfer vector comprising the encapsidation signal and the gene(s) of interest or a cloning site for inserting the gene(s) of interest;
   and
3. a vector containing sequences encoding an element necessary for targeting the viral particle to the intended recipient cell, preferably the gene encoding the G glycoprotein of the vesicular stomatis virus (VSV-G) or amphotrophic MuLV or lentiviral *envs.*

Using the CMV promoter or other strong, high efficiency, promoter instead of the HIV-1 LTR promoter in the packaging vector, high expression of *gag, pol,* or *gag*/*pol* can be achieved in the total absence of any other viral protein. The exchange of the HIV-1 LTR promoter with other promoters is beneficial in the packaging vector or other vectors if constitutive expression is desirable and also for expression in other mammalian cells, such as mouse cells, in which the HIV-1 promoter is weak. Vectors containing the sequences of the disclosure can be used for the Rev independent production of HIV-1 Gag/Pol, HIV-1 Gag, HIV-1 Pol, and SIV Gag proteins. In certain embodiments, the presence of heterologous promoters will also be desired in the transfer vector and the envelope encoding vector, when such vectors are used.

The gene(s) of interest are chosen according to the effect sought to be achieved. For gene therapy purposes there will be at least one therapeutic gene encoding a gene product which is active against the condition it is desired to treat or prevent. Alternatively or additionally, there may be a gene which acts as a marker by encoding a detectable product. Therapeutic genes may encode, for example, an anti-sense RNA, a ribozyme, a transdominant negative mutant of a target protein, a toxin, a conditional toxin, an antigen that induces antibodies or helper T-cells or cytotoxic T-cells, a single chain antibody or a tumor suppresser protein. See, e.g., WO 98/17816.

An even more extensive list of genes of interest for use in lentiviral vectors is described, e.g., in WO 99/04026 on page 10, line 20 to page 12, line 7. Table 2 of Klimatcheva et al. (1999) also provides a list of disorders and target cells for gene therapy, as well as a number of lentiviral vectors used by others. This list includes genetic/metabolic deficiencies, viral infection and cancer. Inherited genetic defects such as adenosine deaminase deficiency, familial hypercholesterolemia, cystic fibrosis, mucopolysaccharidosis type VII, types I and II diabetes, classical phenylketonuria and Gaucher disease are diseases which are listed as being possible to overcome by lentiviral vector-mediated gene therapy because they constitute single-gene deficiencies for which the involved genes are known. Viral diseases are also listed as constituting appropriate targets for lentiviral gene delivery. In particular, a number of gene therapy approaches have been proposed for the treatment of HIV infection and, for some of these strategies, phase I studies have recently begun in humans. The article states that preliminary studies have dealt with defective murine oncoviruses for delivery of anti-sense RNAs, ribozymes and trans-dominant proteins against HIV replication.

In any of the vectors, but preferably in the transfer vector, an inserted gene could have an internal ribosomal entry site (IRES), e.g., from picornaviral RNA. An IRES will be used in circumstances that one wants to express two proteins from the same promoter. For example one protein of interest and a marker gene, e.g., green fluorescent protein (GFP) or a marker gene and a drug resistance gene (e.g. the firefly luciferase gene and neomycin phosphotransferase gene) as described on p. 58 of WO 99/04026, for example. Using an IRES the expression of the two proteins is coordinated. A further gene or genes may also be present under the control of a separate promoter. Such a gene may encode for example a selectable marker, or a further therapeutic agent which may be among the therapeutic agents listed above. Expression of this gene may be constitutive; in the case of a selectable marker this may be useful for selecting successfully transfected packaging cells, or packaging cells which are producing particularly high titers of the retroviral vector particles. Alternatively or additionally, the selectable marker may be useful for selecting cells which have been successfully infected with the lentiviral vector and have the provirus integrated into their own genome.

One way of performing gene therapy is to extract cells from a patient, infect the extracted cells with a lentiviral vector and reintroduce the cells back into the patient. A selectable marker may be used to provide a means for enriching for infected or transduced cells or positively selecting for only those cells which have been infected or transduced, before reintroducing the cells into the patient. This procedure may increase the chances of success of the therapy. Selectable markers may be for instance drug resistance genes, metabolic enzyme genes, or any other selectable markers known in the art. Typical selection genes encode proteins that confer resistance to antibiotics and other toxic substances, e.g., histidinol, puromycin, hygromycin, neomycin, methotrexate etc. and cell surface markers.

However, it will be evident that for many gene therapy applications of lentiviral vectors, selection for expression of a marker gene may not be possible or necessary. Indeed expression of a selection marker, while convenient for *in vitro* studies, could be deleterious *in vivo* because of the inappropriate induction of cytotoxic T lymphocytes (CTLs) directed against the foreign marker protein. Also, it is possible that for *in vivo* applications, vectors without any internal promoters will be preferable. The presence of internal promoters can affect for example the transduction titres obtainable from a packaging cell line and the stability of the integrated vector. Thus, single transcription unit vectors, which may be bi-cistronic or poly-cistronic, coding for one or two or more therapeutic genes, may be the preferred vector designed for use *in vivo.* See, e.g., WO 98/17816.

Suitable host or producer cells for use in the invention are well known in the art. May lentiviruses have already been split into replication defective genomes and packaging components. For those which have not the technology is available for doing so. The producer cell encodes the viral components not encoded by the vector genome such as the Gag, Pol and Env proteins. The *gag, pol* and *env* genes may be introduced into the producer cell transiently, or may be stably integrated into the cell genome to give a packaging cell line. The lentiviral vector genome is then introduced into the packaging cell line by transfection or transduction to create a stable cell line that has all of the DNA sequences required to produce a lentiviral vector particle. Another approach is to introduce the different DNA sequences that are required to produce lentiviral vector particle, e.g., the *env* coding constrict, the *gag-pol* coding construct and the transfer construct into the cell simultaneously by transient triple transfection.

Target cells identified by Klimatcheva et al. (1999), and the references cited therein, include airway epithelial cells for cystic fibrosis; retinal photoreceptor cells for retinitis pigmentosa; progenitors for red blood cells, macrophages, and lymphocytes for hematopoietic disorders, sickle cell anemia, β-thalassemia, lysosomal storage disorders, mucopolysaccharidoses, and severe combined immunodeficiency syndrome; bone marrow cells and macrophages for Gaucher's disease; liver cells for familial hypercholesterolaemia; T-lymphocytes and macrophages for HIV infection; brain tissue, neurons, and glial cells for neurodegenerative diseases such as Parkinson's and Alzheimer's diseases; endothelial cells and cardiac myocytes for cardiovascular diseases; and cancer cells in various tissues (e.g. liver or brain) for cancer. Target cells for other diseases would be apparent to one of skill in the art.

Vaccines and pharmaceutical compositions comprising at least one of the nucleic acid sequences, vectors, vector systems, or transduced or transfected host cells of the disclosure and a physiologically acceptable carrier are also disclosued

As used herein, the term "transduction" generally refers to the transfer of genetic material into the host via infection, e.g., in this case by the lentiviral vector. The term "transfection" generally refers to the transfer of isolated genetic material into cells via the use of specific transfection agents (e.g., calcium phosphate, DEAE Dextran, lipid formulations, gold particles, and other microparticles) that cross the cytoplasmic membrane and deliver some of the genetic material into the cell nucleus.

Systems similar to those described herein can be produced using elements of lentiviruses in addition to the HIV and/or SIV genes described herein.

### Pharmaceutical Compositions

The pharmaceutical compositions of the disclosure contain a pharmaceutically and/or therapeutically effective amount of at least one nucleic acid construct, vector, vector system, viral particle/virus stock, or host cell (i.e., agents) of the disclosure. In one embodiment of the disclosure, the effective amount of an agent of the invention per unit dose is an amount sufficient to cause the detectable expression of the gene of interest. In another embodiment of the disclosure, the effective amount of agent per unit dose is an amount sufficient to prevent, treat or protect against deleterious effects (including severity, duration, or extent of symptoms) of the condition being treated. The effective amount of agent per unit dose depends, among other things, on the species of mammal inoculated, the body weight of the mammal and the chosen inoculation regimen, as is well known in the art. The dosage of the therapeutic agents which will be most suitable for prophylaxis or treatment will also vary with the form of administration, the particular agent chosen and the physiological characteristics of the particular patient under treatment. The dose is administered at least once. Subsequent doses may be administered as indicated.

To monitor the response of individuals administered the compositions of the disclosure, mRNA or protein expression levels may be determined. In many instances it will be sufficient to assess the expression level in serum or plasma obtained from such an individual. Decisions as to whether to administer another dose or to change the amount of the composition administered to the individual may be at least partially based on the expression levels.

The term "unit dose" as it pertains to the inocula refers to physically discrete units suitable as unitary dosages for mammals, each unit containing a predetermined quantity of active material (e.g., nucleic acid, virus stock or host cell) calculated to produce the desired effect in association with the required diluent. The titers of the virus stocks to be administered to a cell or animal will depend on the application and on type of delivery (e.g., *in vivo* or *ex vivo*). The virus stocks can be concentrated using methods such as centrifugation. The titers to be administered *ex vivo* are preferably in the range of 0.001 to I infectious unit /cell. Another method of generating viral stocks is to cocultivate stable cell lines expressing the virus with the target cells. This method has been used to achieve better results when using traditional retroviral vectors because the cells can be infected over a longer period of time and they have the chance to be infected with multiple copies of the vector.

For *in vivo* administration of nucleic acid constructs, vectors, vector systems, virus stocks, or cells which have been transduced or transfected *ex vivo,* the dose is to be determined by dose escalation, with the upper dose being limited by the onset of unacceptable adverse effects. Preliminary starting doses may be extrapolated from experiments using lentiviral vectors in animal models, by methods known in the art, or may be extrapolated from comparisons with known retroviral (e.g., adenoviral) doses. Generally, small dosages will be used initially and, if necessary, will be increased by small increments until the optimum effect under the circumstances is reached. Exemplary dosages are within the range of 10⁸ up to approximately 5 x 10¹⁵ particles.

Inocula are typically prepared as a solution in a physiologically acceptable carrier such as saline, phosphate-buffered saline and the like to form an aqueous pharmaceutical composition.

The agents of the disclosure are generally administered with a physiologically acceptable carrier or vehicle therefor. A physiologically acceptable carrier is one that does not cause an adverse physical reaction upon administration and one in which the nucleic acids are sufficiently soluble to retain their activity to deliver a pharmaceutically or therapeutically effective amount of the compound. The pharmaceutically or therapeutically effective amount and method of administration of an agent of the disclosure may vary based on the individual patient, the indication being treated and other criteria evident to one of ordinary skill in the art. A therapeutically effective amount of a nucleic acid is one sufficient to prevent, or attenuate the severity, extent or duration of the deleterious effects of the condition being treated without causing significant adverse side effects. The route(s) of administration useful in a particular application are apparent to one or ordinary skill in the art.

Routes of administration of the agents of the disclosure include, but are not limited to, parenteral, and direct injection into an affected site. Parenteral routes of administration include but are not limited to intravenous, intramuscular, intraperitoneal and subcutaneous. The route of administration of the agents of the disclosure is typically parenteral and is preferably into the bone marrow, into the CSF intramuscular, subcutaneous, intradermal, intraocular, intracranial, intranasal, and the like. See, e.g., WO 99/04026 for examples of formulations and routes of administration.

The present invention includes compositions of the agents described above, suitable for parenteral administration including, but not limited to, pharmaceutically acceptable sterile isotonic solutions. Such solutions include, but are not limited to, saline and phosphate buffered saline for nasal, intravenous, intramuscular, intraperitoneal, subcutaneous or direct injection into a joint or other area.

In providing the agents of the present disclosure to a recipient mammal, preferably a human, the dosage administered will vary depending upon such factors as the mammal's age, weight, height, sex, general medical condition, previous medical history and the like.

The administration of the pharmaceutical compositions of the disclosure may be for either "prophylactic" or "therapeutic" purpose. When provided prophylactically, the compositions are provided in advance of any symptom. The prophylactic administration of the composition serves to prevent or ameliorate any subsequent deleterious effects (including severity, duration, or extent of symptoms) of the condition being treated. When provided therapeutically, the composition is provided at (or shortly after) the onset of a symptom of the condition being treated.

For all therapeutic, prophylactic and diagnostic uses, one or more of the agents of the disclosure, as well as antibodies and other necessary reagents and appropriate devices and accessories, may be provided in kit form so as to be readily available and easily used.

Where immunoassays are involved, such kits may contain a solid support, such as a membrane (e.g., nitrocellulose), a bead, sphere, test tube, rod, and so forth, to which a receptor such as an antibody specific for the target molecule will bind. Such kits can also include a second receptor, such as a labeled antibody. Such kits can be used for sandwich assays to detect toxins. Kits for competitive assays are also envisioned.

### VI. INDUSTRIAL APPLICABILITY

Mutated genes of this disclosure can be expressed in the native host cell or organism or in a different cell or organism. The mutated genes can be introduced into a vector such as a plasmid, cosmid, phage, virus or mini-chromosome and inserted into a host cell or organism by methods well known in the art. In general, the mutated genes or constructs containing these mutated genes can be utilized in any cell, either eukaryotic or prokaryotic, including mammalian cells (e.g., human (e.g., HeLa), monkey (e.g., Cos), rabbit (e.g., rabbit reticulocytes), rat, hamster (e.g., CHO and baby hamster kidney cells) or mouse cells (e.g., L cells), plant cells, yeast cells, insect cells or bacterial cells (e.g., E. coli). The vectors which can be utilized to clone and/or express these mutated genes are the vectors which are capable of replicating and/or expressing the mutated genes in the host cell in which the mutated genes are desired to be replicated and/or expressed. See, e.g., F. Ausubel et al., Current Protocols in Molecular Biology, Greene Publishing Associates and Wiley-Interscience (1992) and Sambrook et al. (1989) for examples of appropriate vectors for various types of host cells. The native promoters for such genes can be replaced with strong promoters compatible with the host into which the gene is inserted. These promoters may be inducible. The host cells containing these mutated genes can be used to express large amounts of the protein useful in enzyme preparations, pharmaceuticals, diagnostic reagents, vaccines and therapeutics.

Mutated genes or constructs containing the mutated genes may also be used for in-vivo or in-vitro gene therapy. For example, a mutated gene will produce an mRNA in situ to ultimately increase the amount of protein expressed. Such gene include viral genes and/or cellular genes. Such a mutated gene is expected to be useful, for example, in the development of a vaccine and/or genetic therapy.

The constructs and/or proteins made by using constructs encoding the mutated gag, env, and pol genes could be used, for example, in the production of diagnostic reagents, vaccines and therapies for AIDS and AIDS related diseases. The inhibitory/instability elements in the HIV-1 gag gene may be involved in the establishment of a state of low virus production in the host. HIV-1 and the other lentiviruses cause chronic active infections that are not cleared by the immune system. It is possible that complete removal of the inhibitory/instability sequence elements from the lentiviral genome would result in constitutive expression. This could prevent the virus from establishing a latent infection and escaping immune system surveillance. The success in increasing expression of the entire *gag*/*pol* gene by climinating the inhibitory sequence element suggests that one could produce lentiviruses without any negative elements. Such lentiviruses could provide a novel approach towards attenuated vaccines.

For example, vectors expressing high levels of Gag can be used in immunotherapy and immunoprophylaxis, after expression in humans. Such vectors include retroviral vectors and also include direct injection of DNA into muscle cells or other receptive cells, resulting in the efficient expression of gag, using the technology described, for example, in Wolff et al., Science 247:1465-1468 (1990), Wolff et al., Human Molecular Genetics 1(6):363-369 (1992) and Ulmer et al., Science 259:1745-1749 (1993). Further, the gag constructs could be used in transdominant inhibition of HIV expression after the introduction into humans. For this application, for example, appropriate vectors or DNA molecules expressing high levels of p55^{gag} or p37^{gag} would be modified to generate transdominant gag mutants, as described, for example, in Trono et al., Cell 59:113-120 (1989). The vectors would be introduced into humans, resulting in the inhibition of HIV production due to the combined mechanisms of gag transdominant inhibition and of immunostimulation by the produced gag protein. In addition, the gag constructs of the disclosure could be used in the generation of new retroviral vectors based on the expression of lentiviral gag proteins. Lentiviruses have unique characteristics that may allow the targeting and efficient infection of non-dividing cells. Similar applications are expected for vectors expressing high levels of env.

Identification of similar inhibitory/instability elements in SIV indicates that this virus is a convenient model to test these hypotheses. SIV similarly modified could be used in place of HIV in an effort to further minimize the possibility of rearrangement events that would lead to the generation of infectious HIV.

The following examples illustrate certain embodiments of the present invention, but should not be construed as limiting its scope in any way.

### EXAMPLE 1

### Rev-Independent HIV-1 Gag/Pol Molecular Clone

Figure 1 shows the DNA sequence of a Rev-independent HIV-1 *gag*/*pol* molecular clone. This DNA sequence shown encodes the complete Gag and Pol of HIV-1 and can be expressed in a Rev-independent manner when operably linked to a promoter. The Rev-independent gag sequence was described in U.S. Patent Nos. 5,972,596 and 5,965,726 and the Rev-independent *pol* sequence was generated by eliminating the inhibitory/instability sequences using the methods described in U.S. Patent Nos. 5,972,596 and 5,965,726. Others have reportedly made Rev independent *gag* sequences by optimizing codon usage for human cells (see, e.g., WO 98/34640).

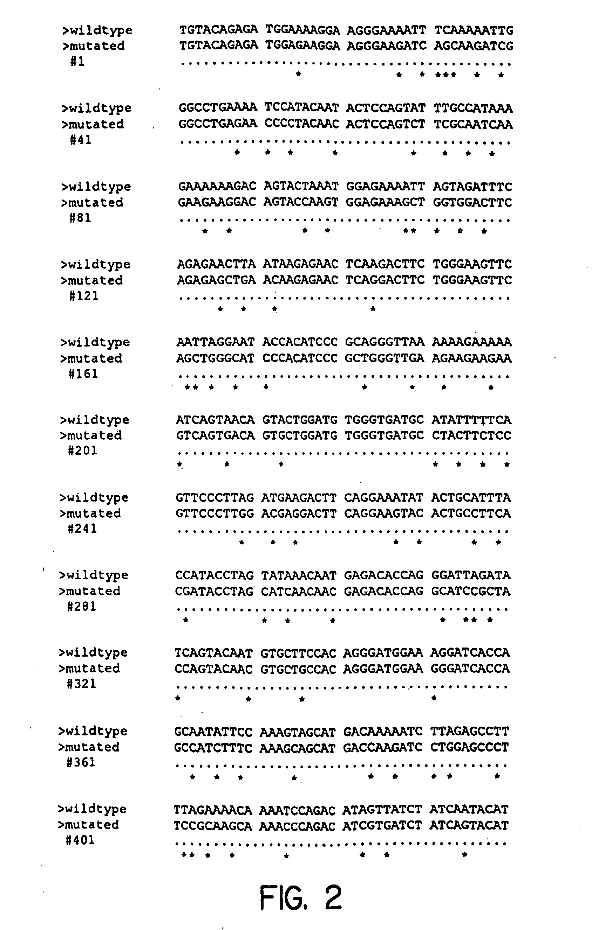

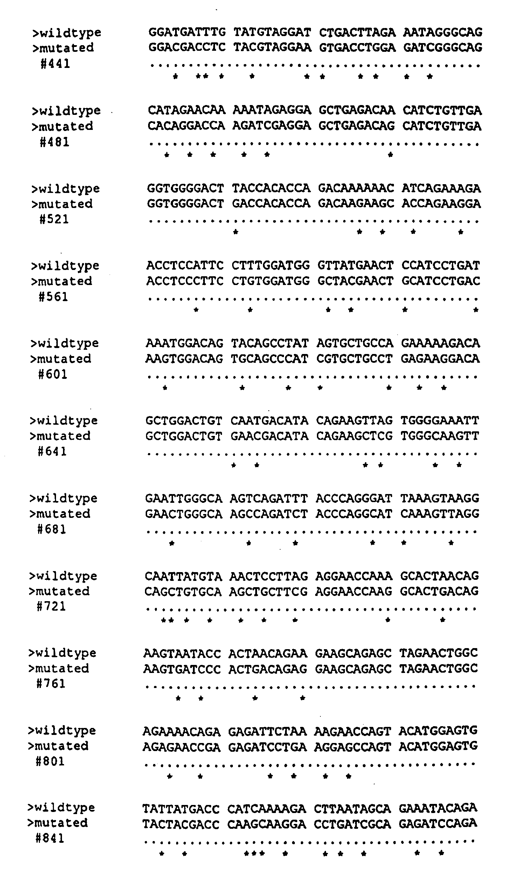

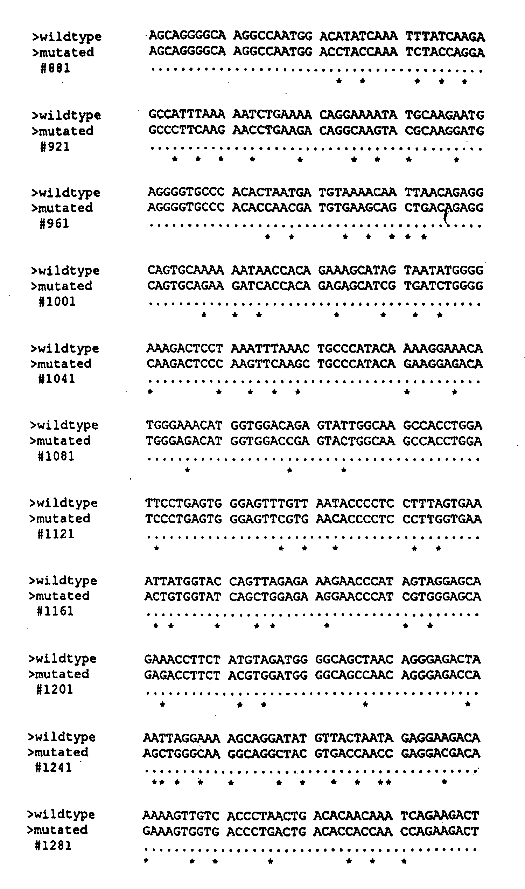

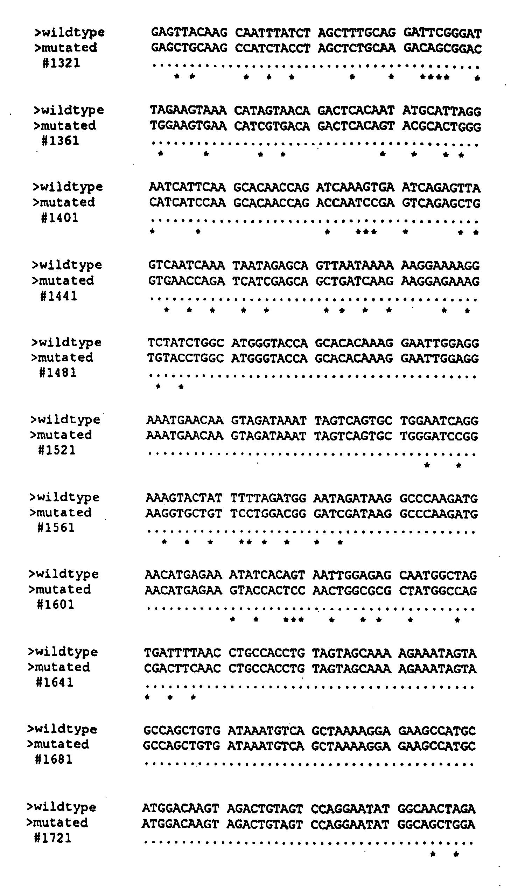

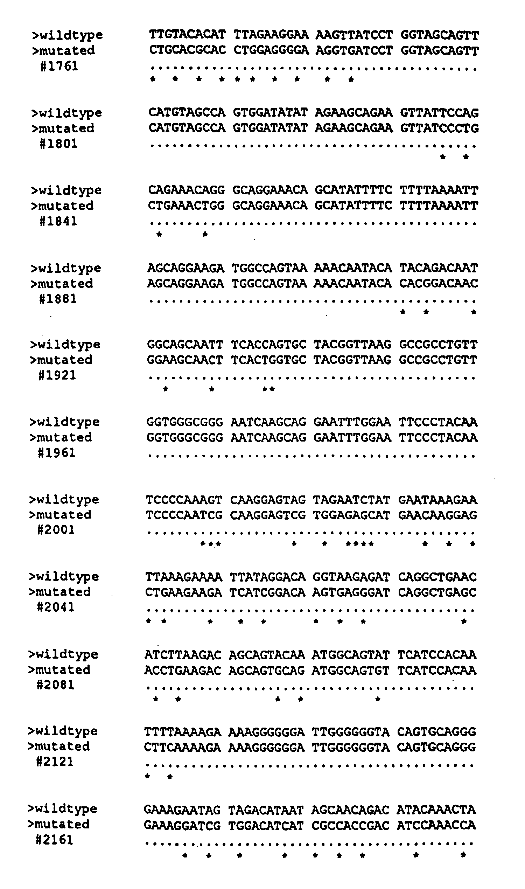

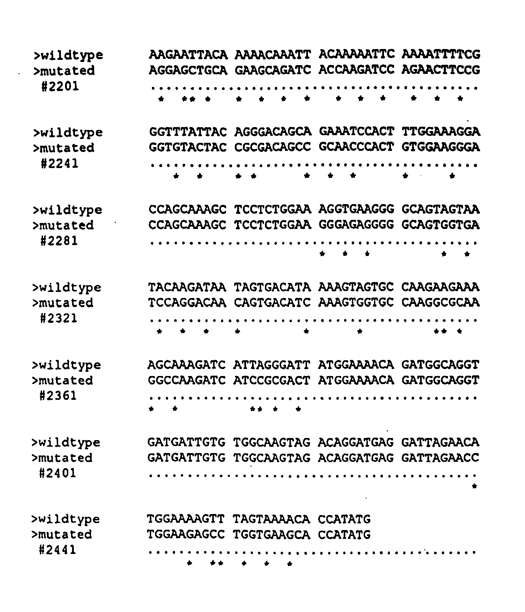

Figure 2 shows an alignment of the sequence of the wild - type and mutated *pol* region in pCMVgagpolBNkan. Position #1 in the Figure is position 2641 in plasmid pCMV gagpolBNkan.

The elimination of INS in *gag, pol* and *env* regions allows the expression of high levels of authentic HIV-1 structural proteins in the absence of the Rev regulatory factor of HIV-1.

### EXAMPLE 2

### Rev-Independent SIV Gag Molecular Clone

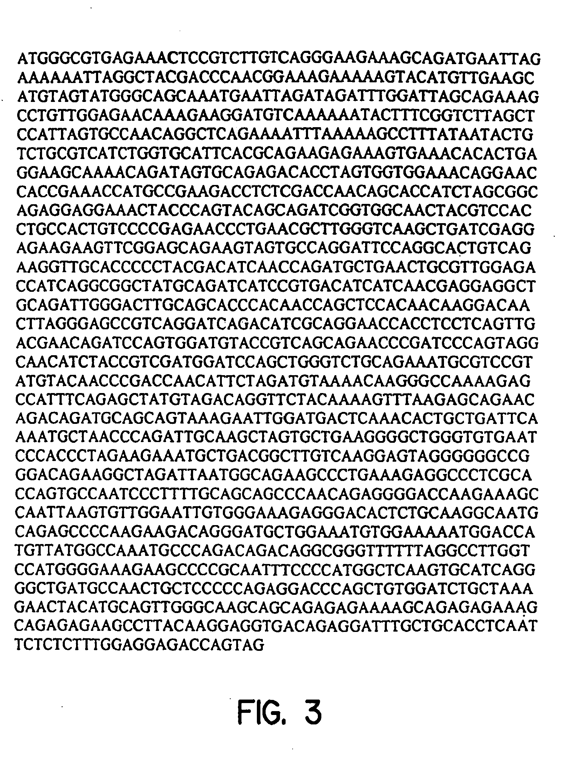

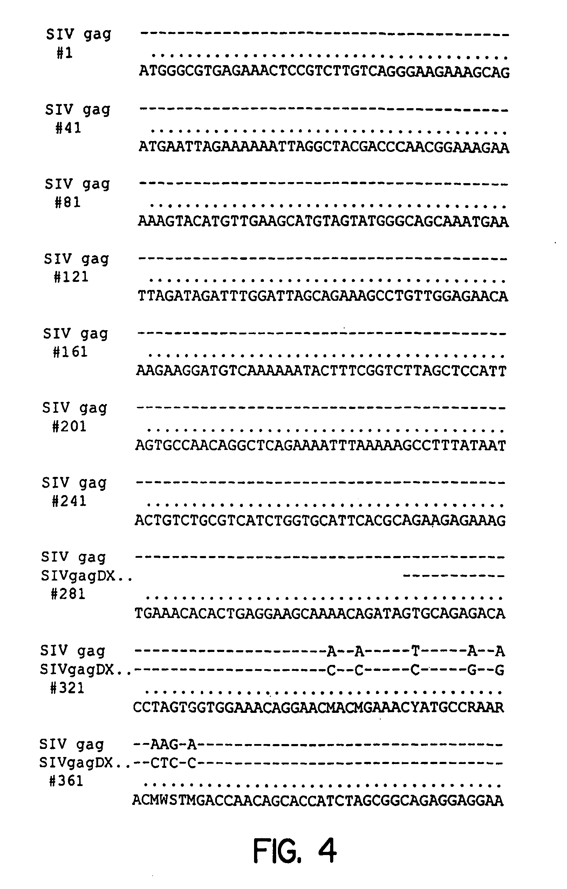

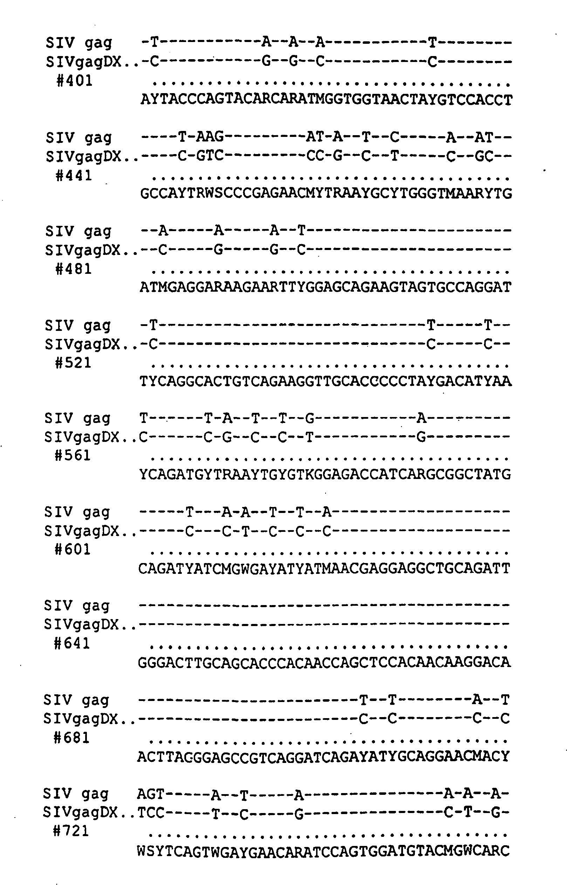

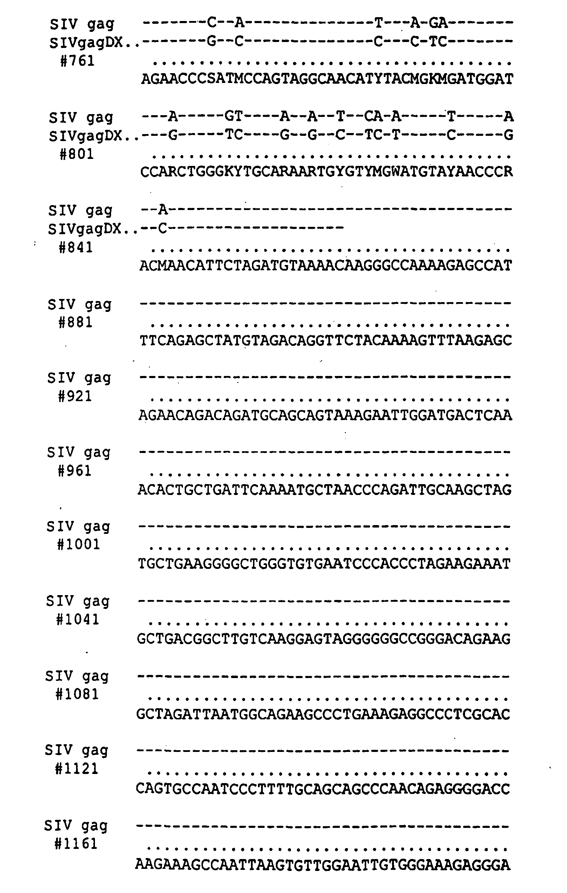

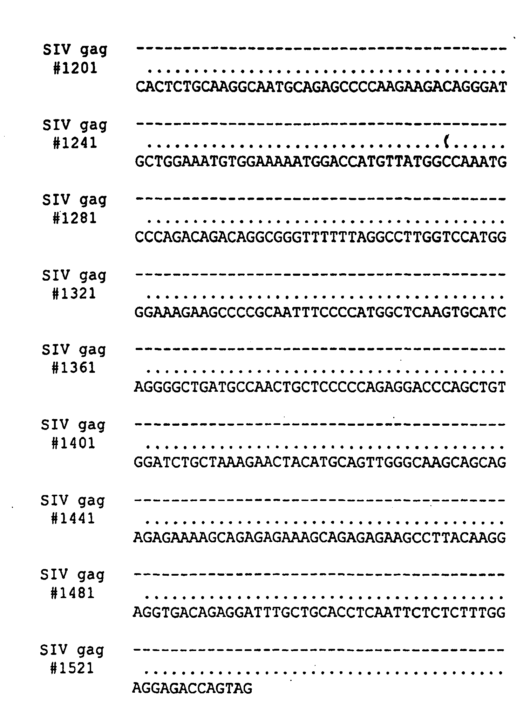

Figure 3 shows the DNA sequence of a Rev-independent SIV *gag* molecular clone, SIVgagDX. Figure 4 shows the comparison of wild type (WT) and mutant (SIVgagDX) sequences. The wild type SIV sequence is from Simian (macaque) immunodeficiency virus isolate 239, clone lambda siv 239-1 (GenBank accession No. M33262).

### EXAMPLE 3

### Rev-Independent SIV Env Molecular Clone

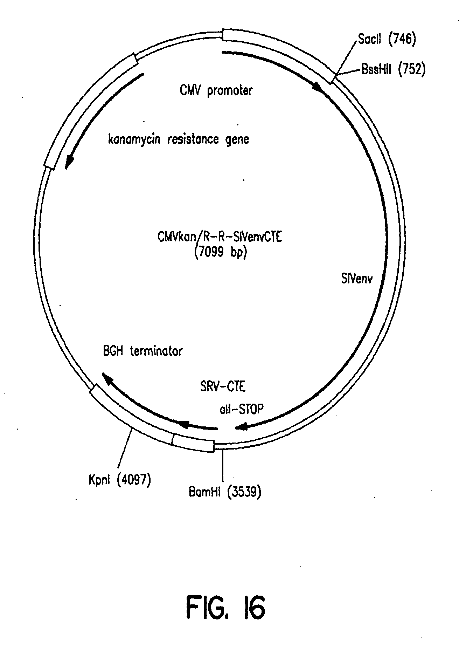

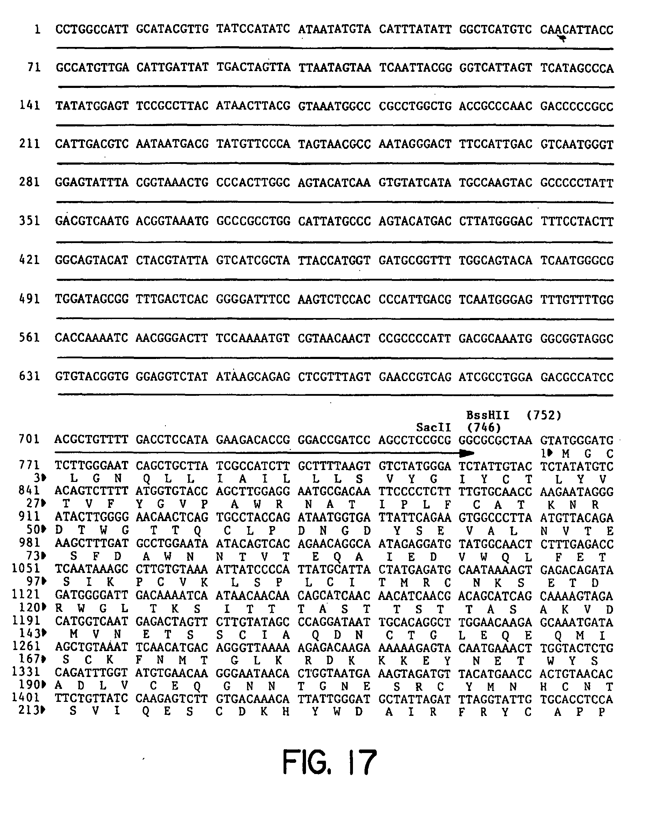

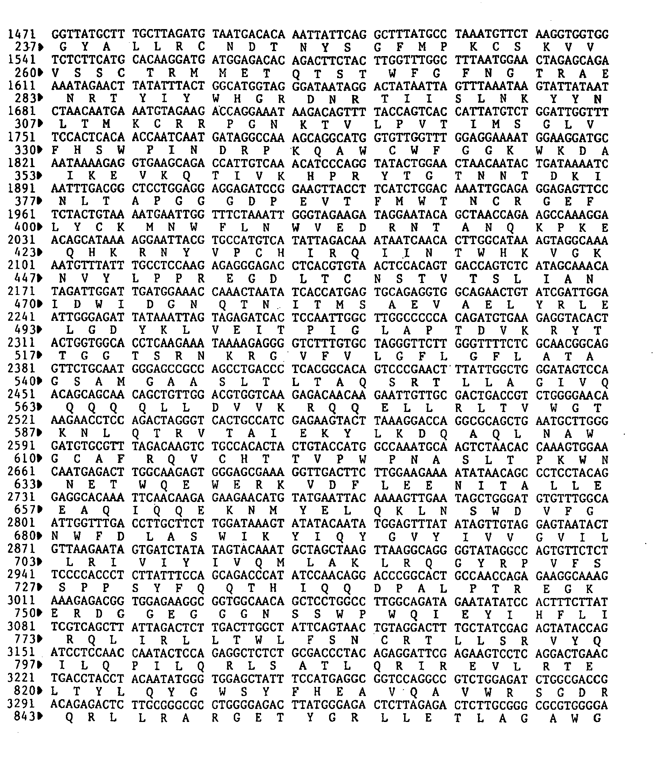

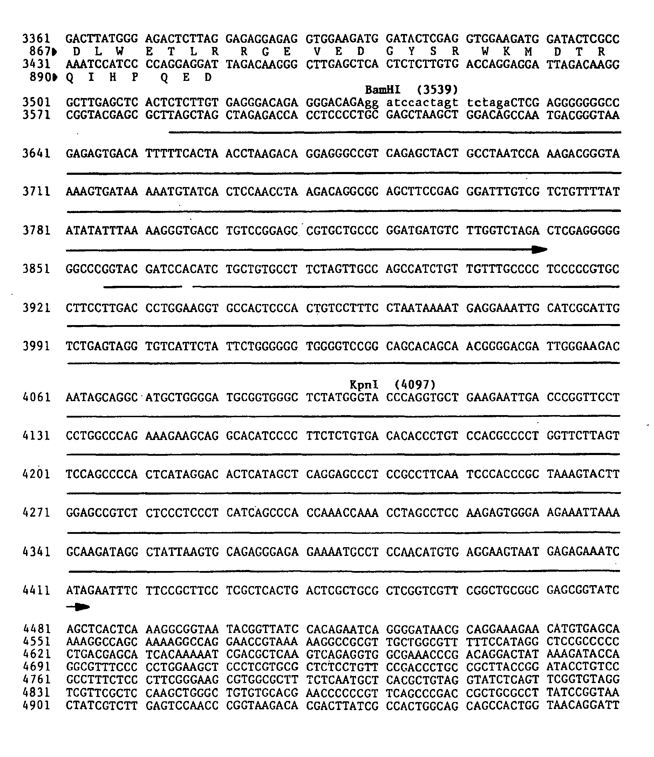

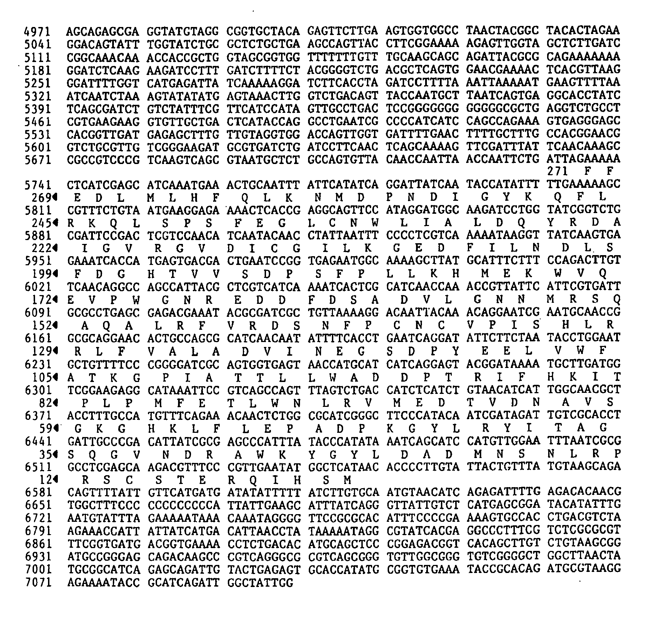

Figure 16 shows a schematic diagram, and figure 17 shows the DNA sequence, of the "env-coding" vector CMVkan/R-R-SIVenvCTE, which is an example of a vector comprising a mutated lentiviral env gene sequence which is capable of being expressed independently of any SIV or HIV regulatory factors. "CMV" denotes the cytomegalovirus promoter; "SRV-CTE" denotes the constitutive transport element (CTE) of Simian Retrovirus Type 1; "all-STOP" denotes a sequence providing translational stops in all three reading frames; "BGH terminator" denotes the bovine growth hormone polyadenylation signal. Other posttranscriptional control elements can be used instead of the indicated SRV-CTE, for example the one described by Pavlakis and Nappi, PCT/US99/11082, filed May 22, 1999, which was published as WO 99/61596 on December 2, 1999 (and which is incorporated herein by reference).

As mentioned previously above, such a vector encoding a lentiviral *env* gene may be used if it is desired that the vector infect CD4⁺T cells. Also as mentioned previously above, the CTE element (i.e., the SRV-CTE element in the case of vector CMVkan/R-R-SIVenvCTE), can be replaced with another post-transcriptional control element, such as the Pavlalcis-Nappi element, that is able to replace CTE and HIV RRE/Rev. See Pavlakis and Nappi, PCT/US99/11082, filed May 22, 1999, which was published as WO 99/61596 on December 2, 1999 (and which is incorporated herein by reference).

### EXAMPLE 4

### Lentivirial Vector System

Figure 5 is a schematic of some of the components of a preliminary version of the Rev-independent lentiviral vector system exemplified herein, including a packaging construct and three different transfer vectors which may be used. In the lentiviral system exemplified herein, the packaging construct also contains the gene for kanamycin resistance. The lentiviral system exemplified herein also contains the vector pHCMV-G, which is shown in Figure 5.

In the packaging construct shown in Figure 5, "CMV" denotes the cytomegalovirus promoter, "Gag" denotes the gag gene, which generates components of the virion core, "Pro" denotes "protease" "RT" denotes "reverse transcriptase," 'Int" denotes "integrase" and "BGH poly (A)" denotes the bovine growth hormone polyadenylation signal. The protease, reverse transcriptase, and integrase genes comprise the "pol" gene. In transfer construct 1, "LTR" denotes the HIV "long terminal repeat", which contains a HIV promoter; "mSD" denotes "mutated splice donor site," which is present in the construct so that splicing of the RNA transcript does not occur; "Ψ" denotes the encapsidation signal; "wGA" denotes part of the wild-type *gag* gene which contains sequences believed to be necessary for encapsidation; "X" indicates that the ATG codon of the partial *gag* gene sequence is mutated so that translation of this gene does not occur; "CMV" denotes the cytomegalovirus promoter and luciferase is used as a reporter gene. Luciferase can be replaced with any gene of interest. Another HIV LTR is present at the 3' end of transfer construct 1. Replacement of this LTR in constructs such as the transfer construct 1, 2, or 3 with a promoter-enhancer deleted HIV LTR leads to inactivation of LTR after integration. Transfer construct 2 is similar to transfer construct 1, the difference being that a mutated part of the *gag* gene (denoted "mGa") is used instead of the wild-type part of the *gag* gene. Transfer construct 3 (pm2BCwCNluci) has different mutations at the 5' splice site and has an intact ATG codon so that translation of part of the mutated *gag* gene occurs. Transfer construct 3 also has a 5' CMV promoter instead of a 5' LTR promoter. This construct is expressed independent of the presence of HIV Tat protein. The transfer constructs expressed from the LTR promoter are partially dependent on Tat protein. In 293 cells significant expression can be achieved in the absence of Tat. See, e.g., Valentin et al., Proc. Natl Acad. Sci. USA. 95:8886-91 (1988).

### EXAMPLE 5

### Generation of Packaging Construct pCMV gagpol BNkan

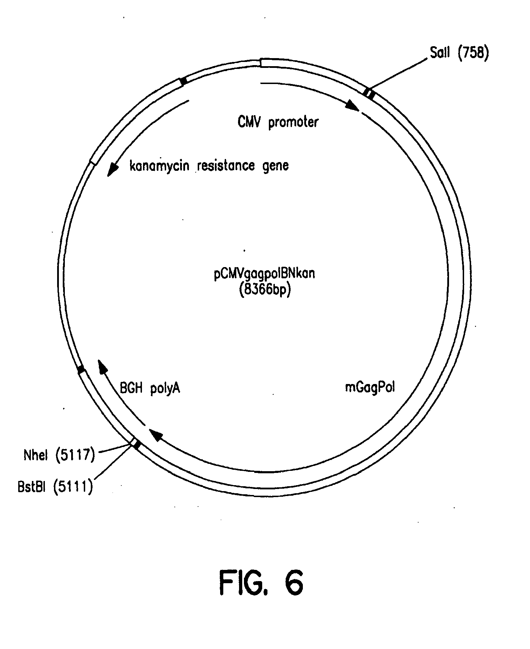

Figure 6 shows a schematic map of the packaging construct pCMV gagpolBNKan. The nucleotide numbering is that of the HXB2R sequence (Genbank accession number K03455 and M38432), where +1 is the start of transcription.

The sequence in HIV-1 *gag*/*pol* region was mutated in order to eliminate all the INS. The fragment from the beginning *of gag* to BsrGI site in *pol,* and the fragment KE [KpnI(3700)- EcoRI(4194)] were previously mutated described in Schneider et al., J Virol. 71: 4892-4903 (1997) and in U.S. Patent Nos. 5,972,596 and 5,965,726.

To generate pCMV gagpolBNkan, three fragments within HIV-1 *pol* region were mutated. They are fragment BP [BsrGI(2207)-PflMI(3032)], fragment PK [PflMI(3032)-KpnI(3700)] and fragment EN [EcoRI(4194)-NdeI(4668)]. Mutagenesis was performed using a modified version of the method described by Ho et al., Gene 77: 51-59 (1989) and DNA shuffling (Zhao and Arnold, Nucl. Acid Res. 25(6), 1307-1308 (1997). Sixteen oligonucleotides extending over the complete sequence of the three fragments were designed. Six oligos corresponded to fragment BP, six to fragment PK, and four to fragment EN (the oligonucleotides ranged from 130 to 195 bases in length; adjacent oligos overlapped by twenty nucleotides). Each fragment was assembled in two steps:
1) PCR; the reaction was carried out in standard *pfu* buffer with 10 pmol of each purified big oligo, 0.2 mM of each dNTPs and 2.5 u *pfu* DNA polymerase enzyme (Stratagene) in a 50 µl final volume. The PCR program was: 3 min 96°C followed by 50 cycles of 1 min 94°C, 1 min 55°C, and 1 min + 5 s/cycle 72°C, ended by 7 min at 72°C. After PCR, the big oligonucleotides were removed from the assembled mutated fragment.
2) The second step was to specifically amplify the assembled products with 30 mer primers located at the 5' and 3' end of each mutated fragment. One microliter of the assembled PCR product was used as template in a 25-cycle PCR reaction with 50 pmol of each primer, 1 x *pfu* buffer, 0.2 mM of each dNTP and 2.5 u *pfu* DNA polymerase in a 50 µl final volume. The PCR program was: 3 min 96°C, 10 cycles of 30 s 94°C, 30 s 55°C, 45 s 72°C, followed by another 14 cycles of 30 s 94°C, 30 s 55°C, 45 s + 20 s/cycle 72°C, and finally 7 min 72°C. This program gave a single PCR product of the correct size. The amplified BP, PK and EN fragments were individually cloned into PCR-script™ vector using PCR-script™ Amp SK(+) Cloning Kit (Stratagene). Clones were randomly selected and sequenced. The correct BP, PK and EN fragments together with fragment KE previously mutated by Schneider et al. were ligated between BsrGI and KpnI site of p55AM1-R5 (which was previously described in Schneider et al., J. Virol. 71: 4892-4903 (1997)) to produce a completely mutated *gagpol* ORF. The new plasmid containing the completely mutated *gaglpol* was named pLTRgagpolBN. BN stands for the modification of the fragment between BsrGI and Ndel. The mutated *gag*/*pol* was then cloned into a CMVkan vector containing the cytomegalovirus major late promoter (GenBank accession no. X17403) and the kanamycin resistance gene, resulting in pCMVgagpolBNkan. The plasmid backbone comes from pVR1332 provided by Vical Inc., and described in Hartikka et al., Hum Gene Ther. 7:1205-17 (1996).

It is understood that different plasmid backbones can be used, e.g., to provide good expression *in vivo,* in the case of DNA injection, for example.

### EXAMPLE 6

### Construction of Transfer Vectors pmBCwCNluci and pmBCmCNluci

The HIV-1 sequence BC, between BssHII (257) and Clal (376), contains the major splice donor site and the encapsidation signal. Six oligos (33 to 46 bases) were designed to introduce mutations on the splice donor site and the AUG start codon of *gag.* The BC fragment was assembled, amplified and sequenced as described in the section concerning the construction of pCMVgagpolBN.

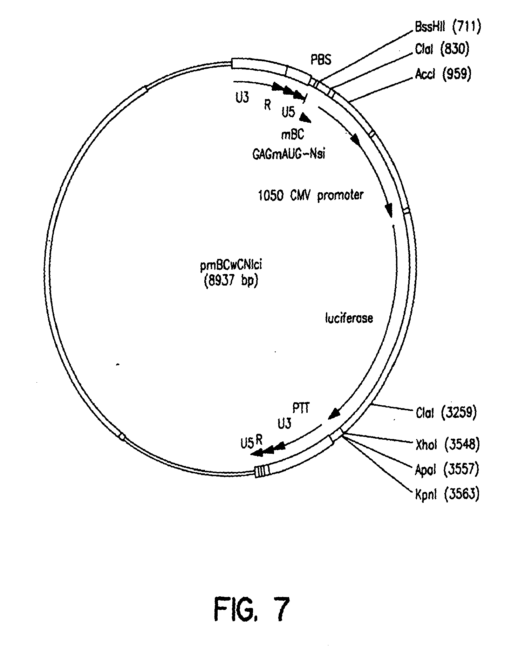

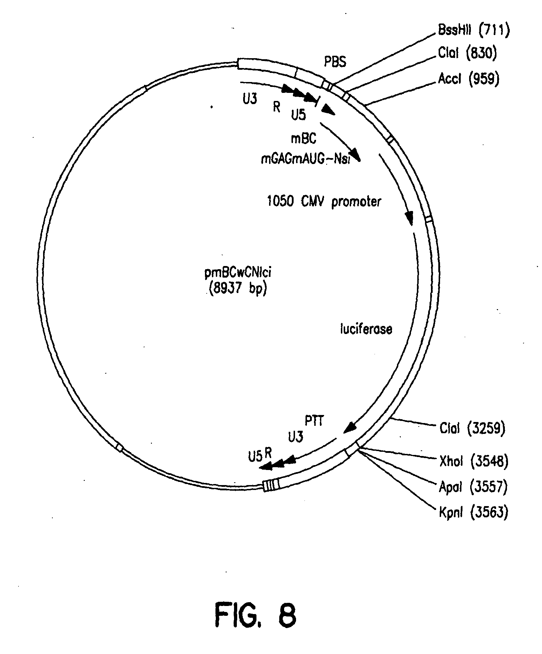

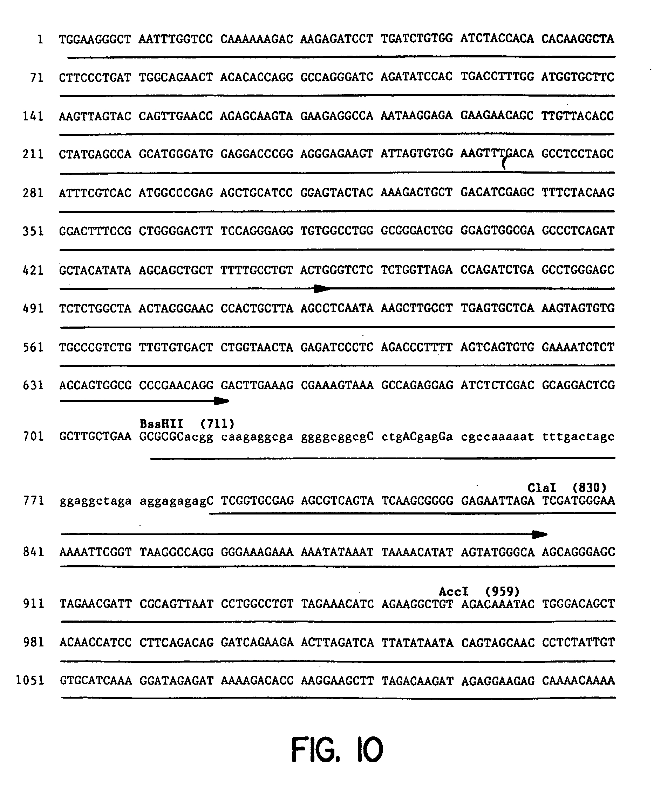

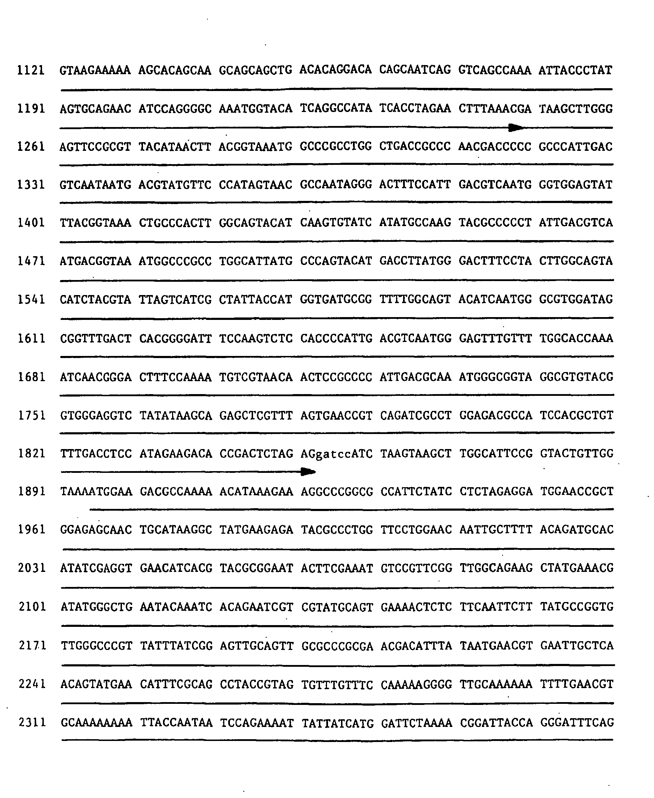

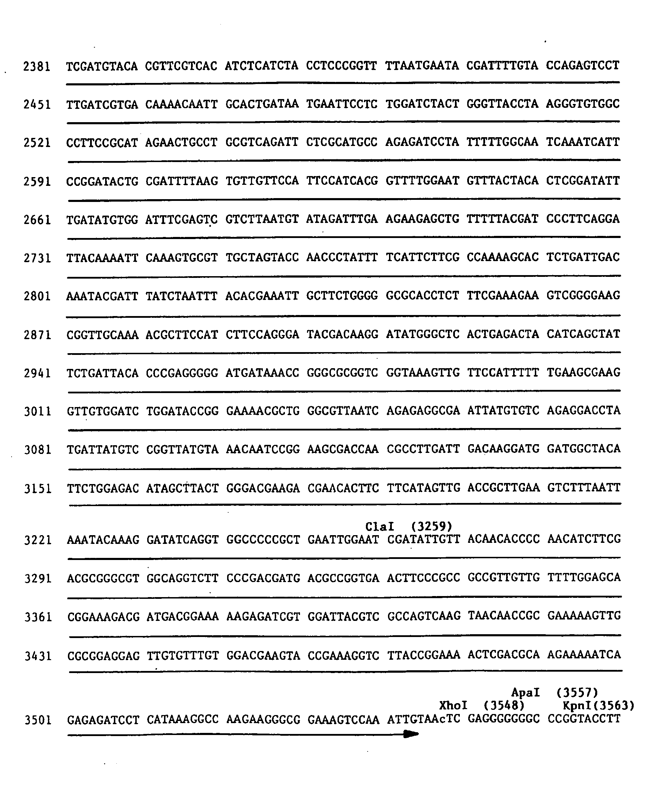

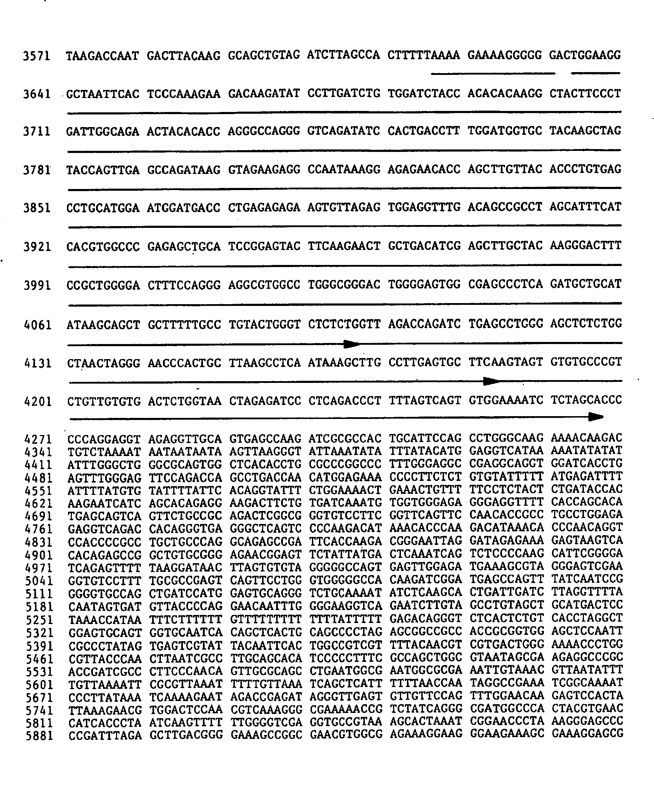

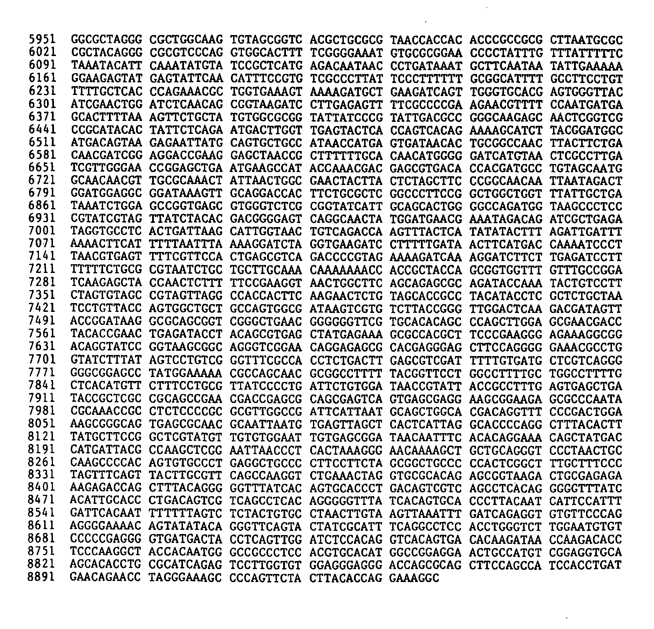

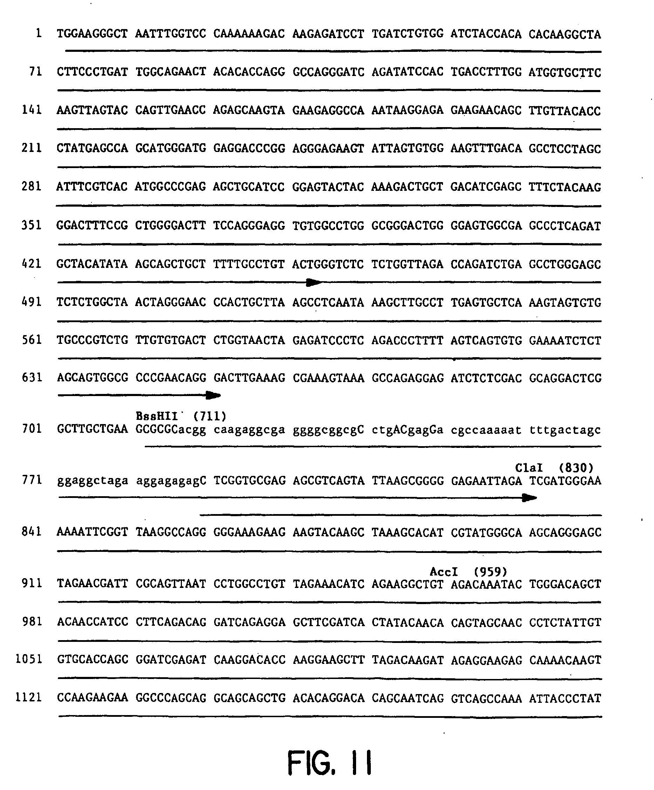

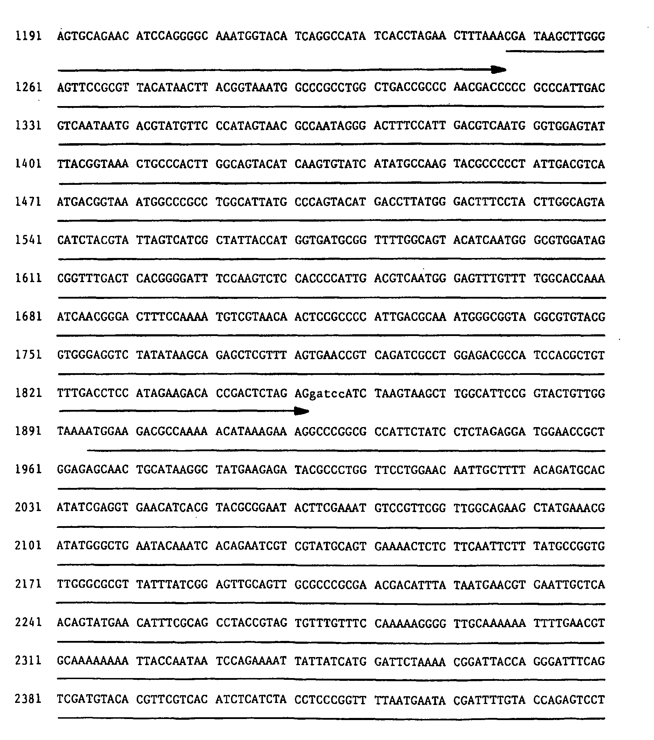

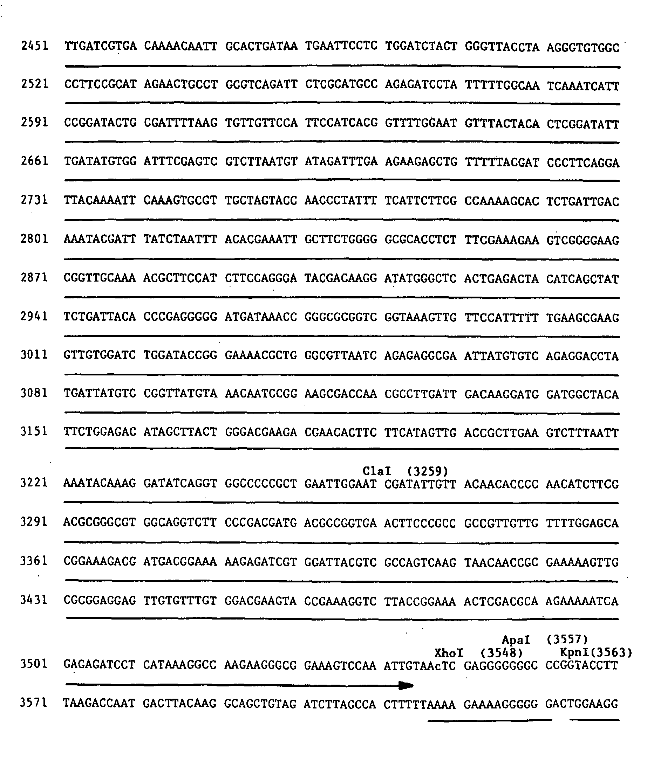

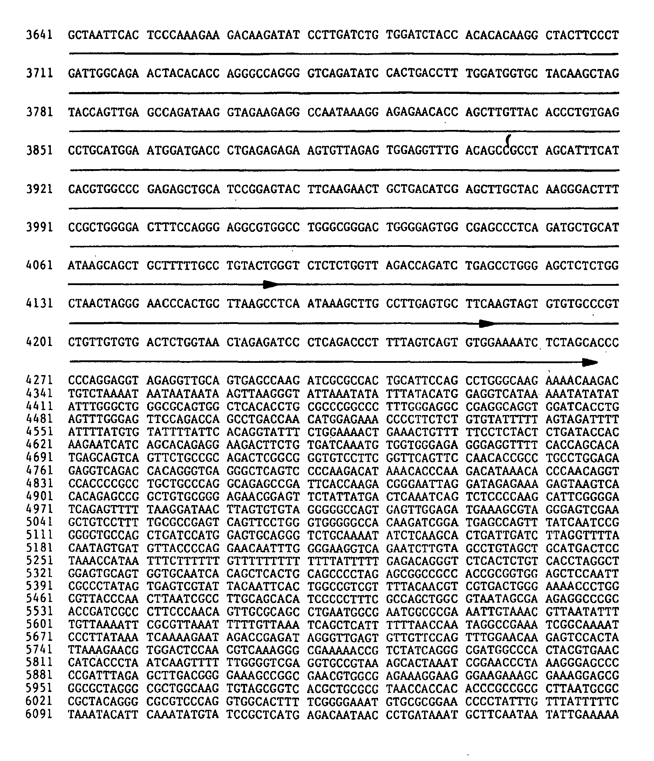

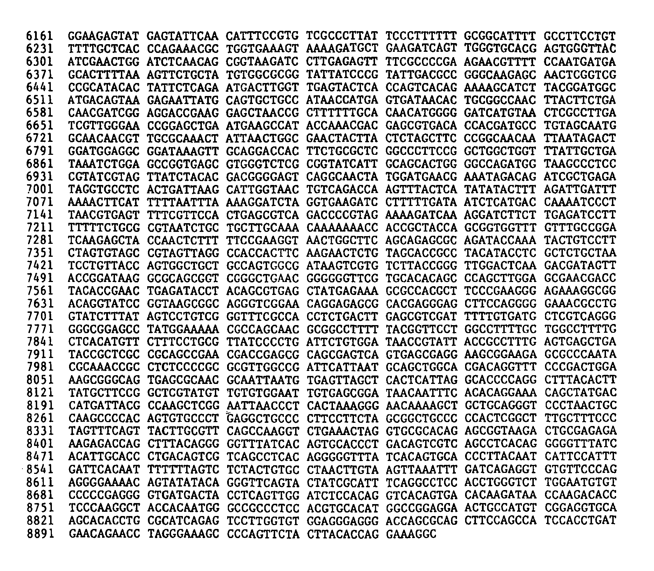

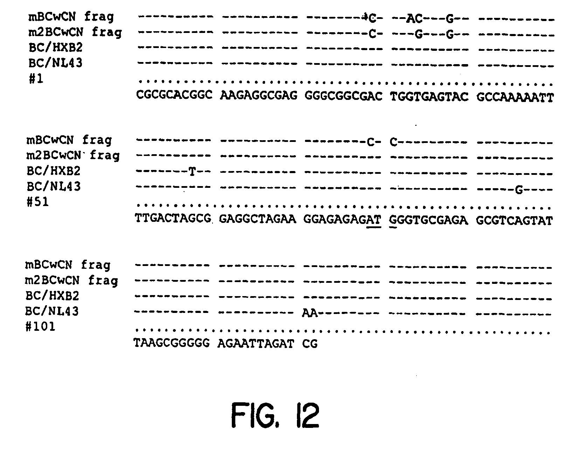

The mutated BC fragment and a fragment of wild type *gag* between Clal (376) and Nsi (793) were placed between the BssHII and Nsi sites of p55RRE (Schneider et al., J. Virol. 71:4892-4903 (1997)) to generate pmBCwCN. In parallel, the fragment between ClaI (376) and NsiI sites of mutated *gag* from p55BM1-10SD+ was used to generate pmBCmCN. (p55BM1-10SD+ is similar to p55BM1-10, which is described in Schneider et al. (1997), but contains in addition the intact splice donor and encapsidation site upstream of gag). The region between Nsil and XhoI containing 3' part of *gag* and RRE in pmBCwCN and pmBCmCN was replaced by a ClaI-XhoI fragment containing CMV promoter and luciferase gene from pHR'-CMVluci (vector from D. Trono) to generate pmBCwCNluci and pmBCmCNluci (which are shown as transfer constructs 1 and 2 in Figure 5, and schematically depicted in Figures 7 and 8, respectively). The sequences of these plasmids are shown in Figures 10 and 11, respectively. Different versions of these plasmids have also been created, by standard procedures, with variations in the region of the encapsidation site, the first splice donor site, and the initiator *gag* AUG. For example, the transfer construct pm2BcwCNIuci (which is shown as transfer construct 3 in Fig. 5) has different mutations in the 5' splice site region and has an intact ATG. A comparison of the sequences in the BssHII-Cla I region of transfer constructs 1 and 2 (mBCwCN frag), transfer construct 3 (m2BCwCN frag), HXB2 and NL43 is shown in Fig. 12.

### EXAMPLE 7

### Preparation of Viral Particles

Lentiviral particles were generated by transient cotransfection of 293 human kidney cells with a combination of three plasmids: pCMVgagpolBNkan, pmBCwCNluci or pmBCmCNluci (transfer vector) and pHCMV-G (Yee et al., Proc. Natl. Acad. Sci., USA, 91:9564-9568 (1994) a plasmid coding for the envelope VSV-G (glycoprotein of vesicular stomatitis virus).

The day before the transfection, 293 cells were plated at a density of 10⁶ cells/plate on a 60 mm plate. Plasmid DNA was transfected by the Ca-phosphate precipitation method in the following proportions: 3 µg packaging construct, 6 µg transfer construct and 100 ng VSV-G encoding construct, pHCMV-G. [Note that the LTR promoter can be expressed in 293 cells in the absence of Tat with a moderate decrease in efficiency. The transfer constructs can be fully Tat independent after replacement of the *LTR* promoter with a CMV (see, *e.g*., transfer construct 3 in Fig. 5) or other promoter in such a way that the mRNA start site is at the beginning of the LTR R region.] In the present experiments for preparation of viral particles 500 ng of a Tat expression plasmid was included in the transfection.

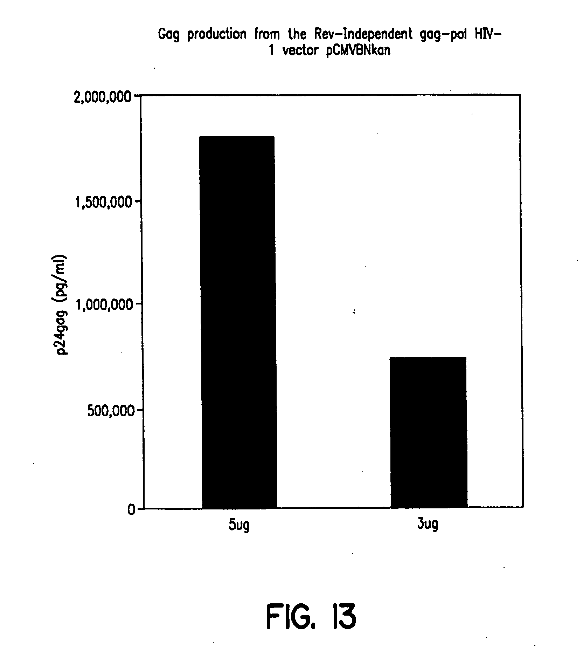

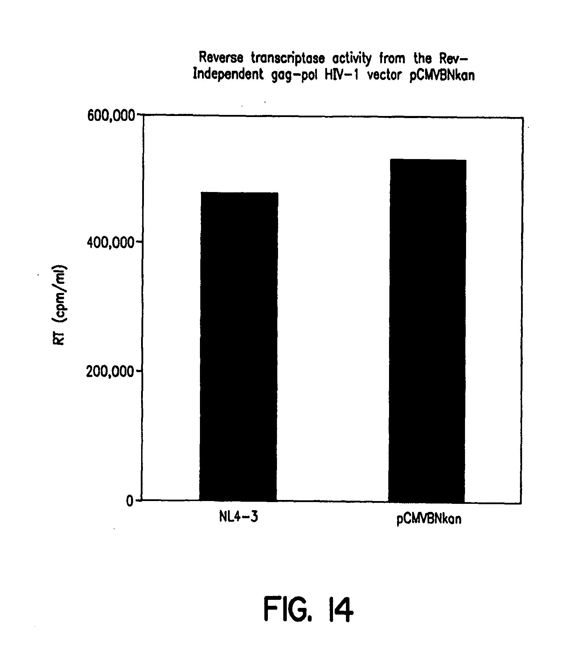

Cells were washed the day after transfection and were kept in DMEM medium for another 48 hours before the supernatants were harvested. Supernatants were spun at 1,200 rpm for 7 mins to eliminate any floating cells. pCMVgagpolBNkan produces high levels of Gag protein that is efficiently released from the cells (Figure 13), and also produces high levels of functional Pol as judged by levels of reverse transcriptase activity similar to those found upon expression of complete HIV-1 (Figure 14).

Supernatants from 293 transfected cells were used to transduce several human cell lines (293, Jurkat, U937) and non-dividing human primary macrophages.

### EXAMPLE 8

### Cell Transduction

Transduction was performed by incubating for 3-4 hours at 37°C the target cells with 1-2 ml of supernatant containing the retroviral vectors. The amount of retroviral vector present in the supernatant was normalized by p24 content (measured by ELISA). Equal amounts of p24 gag protein were used for infection of cells. This way, differences in production of the different preparations was minimized.

The macrophages used for transduction were isolated from the peripheral blood of healthy donors by adherence to plastic. Cells were cultured in RPMI + 20% fetal calf serum (FCS) + 10% human serum (HS). After 1 week, non-adherent cells were washed off with PBS and the macrophages were kept in culture for another 1-2 weeks in the absence of human serum. The cells were washed 2-4 times with PBS before transduction.

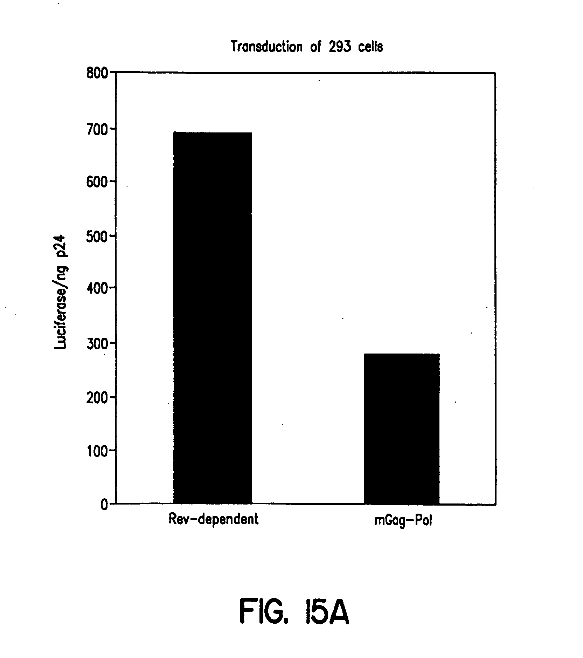

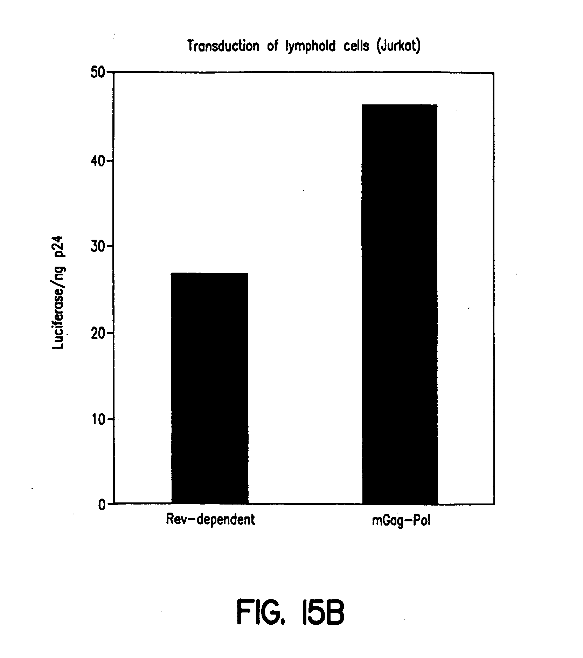

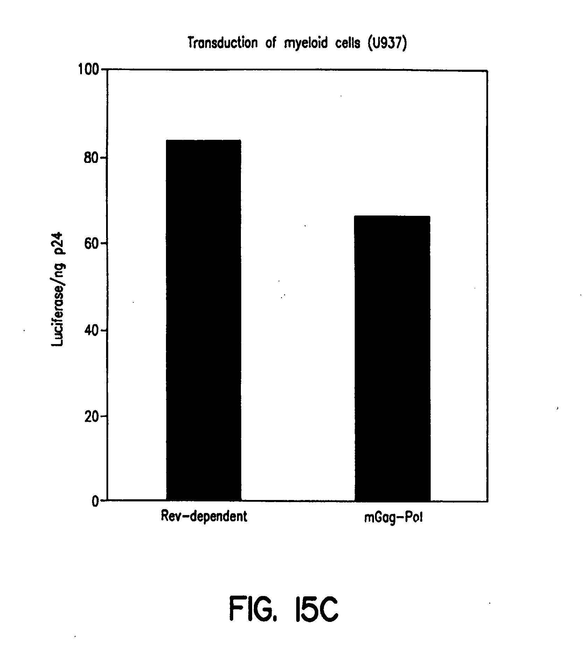

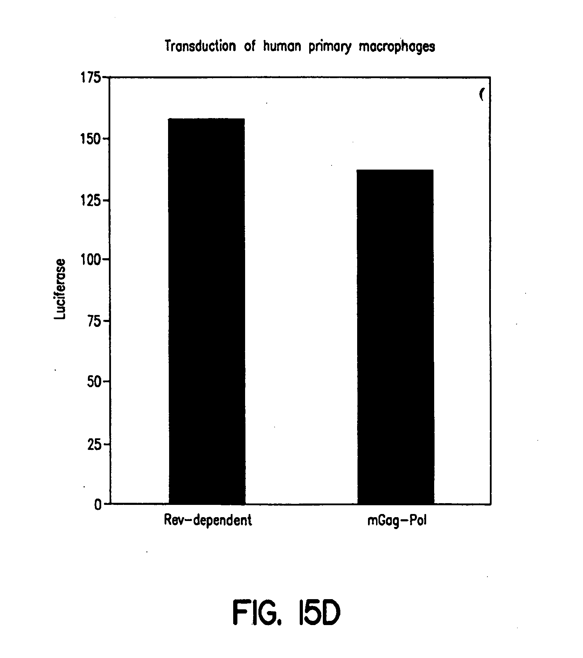

Cells were harvested 48 hours after transduction (seven days for primary macrophages) and the transduction efficiency was determined by measuring luciferase activity in cell extracts from the cultures. The results of the transduction experiments in 293 Jurkat, U937 and primary macrophages are shown in Figure 15A-D. These results demonstrate that Rev-independent *gag*-HIV-1 based retroviral vectors display high transduction efficiency in (A) 293 cells, (B) human lymphoid cells, (C) human myeloid cells (U937), as well as (D) non-dividing cells such as primary human macrophages.

### EXAMPLE 9

### Use Of Nucleic Acids of the Disclosure In Immunoprophylaxis Or Immunotherapy

In postnatal gene therapy, new genetic information has been introduced into tissues by indirect means such as removing target cells from the body, infecting them with viral vectors carrying the new genetic information, and then reimplanting them into the body; or by direct means such as encapsulating formulations of DNA in liposomes; entrapping DNA in proteoliposomes containing viral envelope receptor proteins; calcium phosphate co-precipitating DNA; and coupling DNA to a polylysine-glycoprotein carrier complex. In addition, in vivo infectivity of cloned viral DNA sequences after direct intrahepatic injection with or without formation of calcium phosphate coprecipitates has also been described mRNA sequences containing elements that enhance stability have also been shown to be efficiently translated in Xenopus laevis embryos, with the use of cationic lipid vesicles. See, e.g., J.A. Wolff, et al., Science 247:1465-1468 (1990) and references cited therein.

Recently, it has also been shown that injection of pure RNA or DNA directly into skeletal muscle results in significant expression of genes within the muscle cells. J.A. Wolff, et al., Science 247:1465-1468 (1990). Forcing RNA or DNA introduced into muscle cells by other means such as by particle-acceleration (N. -S. Yang, et al. Proc. Natl. Acad. Sci, USA 87:9568-9572 (1990); S.R. Williams et al., Proc. Natl. Acad. Sci. USA 88:2726-2730 (1991)) or by viral transduction should also allow the DNA or RNA to be stably maintained and expressed. In the experiments reported in Wolff et al., RNA or DNA vectors were used to express reporter genes in mouse skeletal muscle cells, specifically cells of the quadriceps muscles. Protein expression was readily detected and no special delivery system was required for these effects. Polynucleotide expression was also obtained when the composition and volume of the injection fluid and the method of injection were modified from the described protocol. For example, reporter enzyme activity was reported to have been observed with 10 to 100 µl of hypotonic, isotonic, and hypertonic sucrose solutions, Opti-MEM, or sucrose solutions containing 2mM CaCl₂ and also to have been observed when the 10- to 100- µl injections were performed over 20 min. with a pump instead of within 1 min.

Enzymatic activity from the protein encoded by the reporter gene was also detected in abdominal muscle injected with the RNA or DNA vectors, indicating that other muscles can take up and express polynucleotides. Low amounts of reporter enzyme were also detected in other tissues (liver, spleen, skin, lung, brain and blood) injected with the RNA and DNA vectors. Intramuscularly injected plasmid DNA has also been demonstrated to be stably expressed in non-human primate muscle. S. Jiao et al., Hum. Gene Therapy 3:21-33 (1992).

It has been proposed that the direct transfer of genes into human muscle in situ may have several potential clinical applications. Muscle is potentially a suitable tissue for the heterologous expression of a transgene that would modify disease states in which muscle is not primarily involved, in addition to those in which it is. For example, muscle tissue could be used for the heterologous expression of proteins that can immunize, be secreted in the blood, or clear a circulating toxic metabolite. The use of RNA and a tissue that can be repetitively accessed might be useful for a reversible type of gene transfer, administered much like conventional pharmaceutical treatments. See J.A. Wolff, et al., Science 247:1465-1468 (1990) and S. Jiao et al., Hum. Gene Therapy 3:21-33 (1992).

It had been proposed by J.A. Wolff et al., supra, that the intracellular expression of genes encoding antigens might provide alternative approaches to vaccine development. This hypothesis has been supported by a recent report that plasmid DNA encoding influenza A nucleoprotein injected into the quadriceps of BALB/c mice resulted in the generation of influenza A nucleoprotein-specific cytotoxic T lymphocytes (CTLs) and protection from a subsequent challenge with a heterologous strain of influenza A virus, as measured by decreased viral lung titers, inhibition of mass loss, and increased survival. J. B. Ulmer et al., Science 259:1745-1749 (1993).

Therefore, it appears that the direct injection of RNA or DNA vectors encoding the viral antigen can be used for endogenous expression of the antigen to generate the viral antigen for presentation to the immune system without the need for self-replicating agents or adjuvants, resulting in the generation of antigen-specific CTLs and protection from a subsequent challenge with a homologous or heterologous strain of virus.

CTLs in both mice and humans are capable of recognizing epitopes derived from conserved internal viral proteins and are thought to be important in the immune response against viruses. By recognition of epitopes from conserved viral proteins, CTLs may provide cross-strain protection. CTLs specific for conserved viral antigens can respond to different strains of virus, in contrast to antibodies, which are generally strain-specific.

Thus, direct injection of RNA or DNA encoding the viral antigen has the advantage of being without some of the limitations of direct peptide delivery or viral vectors. See J.A. Ulmer et al., supra, and the discussions and references therein). Furthermore, the generation of high-titer antibodies to expressed proteins after injection of DNA indicates that this may be a facile and effective means of making antibody-based vaccines targeted towards conserved or non-conserved antigens, either separately or in combination with CTL vaccines targeted towards conserved antigens. These may also be used with traditional peptide vaccines, for the generation of combination vaccines. Furthermore, because protein expression is maintained after DNA injection, the persistence of B and T cell memory may be enhanced, thereby engendering long-lived humoral and cell-mediated immunity.

### 1. Vectors for the immunoprophylaxis or immunotherapy against HIV-1

The mutated *gag, pol* or *gag*/*pol* sequences will be inserted in expression vectors using a strong constitutive promoter such as CMV or RSV, or an inducible promoter such as HIV-1.

The vector will be introduced into animals or humans in a pharmaceutically acceptable carrier using one of several techniques such as injection of DNA directly into human tissues; electroporation or transfection of the DNA into primary human cells in culture (ex vivo), selection of cells for desired properties and reintroduction of such cells into the body, (said selection can be for the successful homologous recombination of the incoming DNA to an appropriate preselected genomic region); generation of infectious particles containing the *gag* gene, infection of cells *ex vivo* and reintroduction of such cells into the body; or direct infection by said particles in vivo.

Substantial levels of protein will be produced leading to an efficient stimulation of the immune system.

In another embodiment of the invention, the described constructs will be modified to express mutated Gag proteins that are unable to participate in virus particle formation. It is expected that such Gag proteins will stimulate the immune system to the same extent as the wild-type Gag protein, but be unable to contribute to increased HIV-1 production. This modification should result in safer vectors for immunotherapy and immunophrophylaxis.

### EXAMPLE 10

### Inhibition of HIV-1 Expression Using Transdominant (TD)-TD-Gag-TD Rev or Td Gag-Pro-TD Rev Genes

Direct injection of DNA or use of vectors other than retroviral vectors will allow the constitutive high level of trans-dominant Gag (TDgag) in cells. In addition, the approach taken by B.K. Felber et al., Science 239:184-187 (1988) will allow the generation of retroviral vectors, e.g. mouse-derived retroviral vectors, encoding HIV-1 TDgag, which will not interfere with the infection of human cells by the retroviral vectors. In the approach of Felber, et al., supra, it was shown that fragments of the HIV-1 LTR containing the promoter and part of the polyA signal can be incorporated without detrimental effects within mouse retroviral vectors and remain transcriptionally silent. The presence of Tat protein stimulated transcription from the HIV-1 LTR and resulted in the high level expression of genes linked to the HIV-1 LTR.

The generation of hybrid TDgag-TDRev or TDgag-pro-TDRev genes and the introduction of expression vectors in human cells will allow the efficient production of two proteins that will inhibit HIV-1 expression. The incorporation of two TD proteins in the same vector is expected to amplify the effects of each one on viral replication. The use of the HIV-1 promoter in a matter similar to one described in B.K. Felber, et al., supra, will allow high level Gag and Rev expression in infected cells. In the absence of infection, expression will be substantially lower. Alternatively, the use of other strong promoters will allow the constitutive expression of such proteins. This approach could be highly beneficial, because of the production of a highly immunogenic gag, which is not able to participate in the production of infectious virus, but which, in fact, antagonizes such production. This can be used as an efficient immuniprophylactic or immunotherapeutic approach against AIDS.

Examples of trans-dominant mutants are described in Trono et al., Cell 59:112-120 (1989).

### 1. Generation of constructs encoding transdominant Gag mutant proteins

Gag mutant proteins that can act as trans-dominant mutants, as described, for example, in Trono et al., supra, will be generated by modifying vector p37M1-10D or p55M1-13P0 to produce transdominant Gag proteins at high constitutive levels.

The transdominant Gag protein will stimulate the immune system and will inhibit the production of infectious virus, but will not contribute to the production of infectious virus.

The added safety of this approach makes it more acceptable for human application.

### VII. REFERENCES

U.S. Patent No. 5,972,596 issued October 26, 1999 (Pavlakis and Felber)
U.S. Patent No. 5,965,726 issued October 12, 1999 (Pavlakis and Felber)
WO 98/17816 Lentiviral Vectors (Kingsman & Kingsman) (Oxford Biomedica Ltd)
WO 98/34640 (Shiver, J.W., Davies, M-E M., Freed, D.C., Liu, M.A. and Perry,H.C. - Merck & Co., Inc.)
WO 98/46083 Use of Lentiviral Vectors for Antigen Presentation in Dendritic Cells (Wong-Staal, Li; Kan-Mitchell) (Univ. of Cal.)
WO 99/04026 Lentiviral Vectors (Chen, Gasmi, Yee and Jolly) (Chiron)
WO 99/15641 Non-Primate Lentiviral Vectors and Packaging Systems (Poeschia, Looney and Wong-Staal) (Univ. of Cal.)
WO 99/30742 Therapeutic Use of Lentiviral Vectors (Naldini and Song)
WO 99/51754 Infectious Pseudotyped Lentiviral Vectors Lacking Matrix Protein and Uses Thereof (Goettlinger, Reil and Bukovsky) (Dana Farber Cancer Inst Inc)
PCT/US99/11082 Post-Transcriptional Regulatory Elements and Uses Thereof (Pavlakis and Nappi), filed May 22, 1999, published as WO 99/61596 on December 2, 1999
Akkina, R.K., Walton, R.W., Chen, M.L., Li, Q-X, Planelles, V and Chen, LS.Y., "High-efficiency gene transfer into CD34+ cells with a human immunodeficiency virus type 1-based retroviral vector pseudotyped with vesicular stomatitis virus envelope glycoprotein G," J. Virol. 70:2581-2585 (1996)
Amado, R.G. & Chen, LS.Y., "Letinviral vectors—the promise of gene therapy within reach?," Science 285:674-676 (July 1999)
Donahue, R.E., An, D.S., Wersto, R.P., Agricola, B.A., Metzger, M.E. and Chen, LS.Y., "Transplantation of immunoselected CD34+ cells transduced with a EGFP-expressing lentiviral vector in non-human primates," Blood 92(suppl. 1):383b, Abstract #4648.5 (1998)
Fox, J.L., "Researchers wary of fear-based ban on lentivirus gene therapy, "Nature Biotechnology 16:407-408 (1998)
Goldman, M.J., Lee, P.S., Yang, J.S. & Wilson, J.M., "Lentiviral vectors for gene therapy of cystic fibrosis," Hum Gene Ther. 8,2261-2268 (1997)
Hartikka J, Sawdey M, Comefert-Jensen F, Margalith M, Barnhart K, Nolasco M, Vahlsing HL, Meek J, Marquet M, Hobart P, Norman J, and Manthorpe M., "An improved plasmid DNA expression vector for direct injection into skeletal muscle," Hum Gene Ther. 7:1205-17 (1996)
Kafri, T., Blomer, U., Peterson, D.A., Gage, F.H. & Verma, I.M., "Sustained expression of genes delivered directly into liver and muscle by lentiviral vectors," Nat Genet. 17, 314-317 (1997)
Kafri, T., van Praag, H., Ouyang, L., Gage, F.G. and Verma, I.M., "A packaging cell line for lentivirus vectors," J. Virol. 73:576-584 (1999)
Kim, V.N., Mitrophanous, K., Kingsman, S.M., and Kingsman, A.J., "Minimal Requirement for a Lentivirus Vector Based on Human Immunodeficiency Virus Type 1", J. Virol. 72:811-816 (1998)
Klimatcheva, E., Rosenblatt, JD. and Planelles, V., "Lentiviral vectors and gene therapy," Frontiers in Bioscience 4:d481-496 (June 1999)
Miyoshi, H., Takahashi, M., Gage, F.H. & Verma, I.M., "Stable and efficient gene transfer into the retina using an HIV-based lentiviral vector," Proc Natl Acad Sci USA. 94: 10319-10323 (1997)
Miyoshi, H., Blomer,U., Takahashi, M., Gage, F.H., and Verma, I.M., "Development of self-inactivating lentivirus vector," ," J Virol. 72:8150-8157 (1998)
Miyoshi, H., Smith, K.A., Mosier, D.E., Verma, I.M. and Torbett, B.E., "Transduction of human CD34+ cells that mediate long-term engraftment of NOD/SCID mice by HIV vectors," Science 283:682-686 (1999)
Naldini, L., Blomer, U., Gallay, P., Ory, D., Mulligan, R., Gage, F.H., Verma, I.M. & Trono, D., "In vivo gene delivery and stable transduction of nondividing cells by a lentiviral vector," Science. 272, 263-267 (1996)
Naviaux, R.K, Costanzi, E., Haas, M. and Verma, I., "The pCL vector system: rapid production of helper-free, high-titer, recombinant retroviruses," J. Virol. 70:5701-5705 (1996)
Pavlakis, G.N., Schneider, R.; Song, S., Nasioulas, G., Zolotukhin, A., Felber, B.K., Trauger, R., Cox, J., and Manthorpe, M., "Use of simple Rev-independent HIV-1 gag expression vectors in gene therapy and gene vaccine applications," Natl Conf Hum Retroviruses Relat Infect (2nd), Jan 29-Feb 2 (1995); 91.
Poeschla, E.M., Wong-Staal, F. & Looney, D.J., "Efficient transduction of nondividing human cells by feline immunodeficiency virus lentiviral vectors," Nature Med. 4:354-357 (1998)
Qiu, J. T., R. Song, M. Dettenhofer, C. Tian, T. August, B. K. Felber, G. N. Pavlakis and X. F. Yu, "Evaluation of novel human immunodeficiency virus type 1 Gag DNA vaccines for protein expression in mammalian cells and induction of immune responses," J Virol. 73: 9145-52 (Nov. 1999)
Reynolds, P.N. and Curiel, D.T., "Viral vectors show promise in Colorado," Nature Biotechnology 16:422-423 (1998)
Schneider, R., Campbell, M., Nasioulas, G., Felber, B.K., and Pavlakis, G.N., Inactivation of the human immunodeficiency virus type 1 inhibitory elements allows Rev-independent expression of Gag and Gag/protease and particle formation, "J. Virol. 71:4892-4903 (1997)
Schwartz, S., M. Campbell, G. Nasioulas, J. Harrison, B. K. Felber and G. N. Pavlakis, "Mutational inactivation of an inhibitory sequence in human immunodeficiency virus type-1 results in Rev-independent gag expression," J. Virol. 66:7176-7182 (1992)
Shiver, J.W., Yasutomi, Y., Free, D.C., Davies, M.-E., Perry, H.C., Pavlakis, G.N., Letvin, N.L., and Liu, M.A., "DNA Vaccine-Mediated Cellular Immunity Against HIV-1 gag and env", presented at the Conference on Advances in AIDS Vaccine Development: 8th Annual Meeting of the National Cooperative Vaccine Development Groups for AIDS (NCVDGs) from February 11-15, 1996.
Soneoka, Y., Cannon, P.M., Ransdale, E.E., Griffiths, J.C., Romano, G., Kingsman, S.M. and Kingsman, A.J., "A transient three-plasmid expression system for the production of high titer retroviral vectors," Nuc. Acids Res. 23:628-633 (1995).
Srinivasakumar, N., Chazal, N., Helga-Maria, C., Prasad, S., Hammarskjöld, M.-L., and Rekosh, D., "The Effect of Viral Regulatory Protein Expression on Gene Delivery by Human Immunodeficiency Virus Type 1 Vectors Produced in Stable Packaging Cell Lines," J. Virol., 71:5841-5848 (1997)
Sutton, R.E., Wu, H.T., Rigg, R., Bohnlein, E. & Brown, P.O., "Human immunodeficiency virus type 1 vectors efficiently transduce human hematopoietic stem cells," J. Virol. 72, 5781-5788 (1998)
Tabernero, C., A. S. Zolotukhin, J. Bear, R. Schneider, G. Karsenty and B. K. Felber, "Identification of an RNA sequence within an intracisternal-A particle element able to replace Rev-mediated posttranscriptional regulation of human immunodeficiency virus type 1," J Virol. 71:95-101 (1997).. (see also my email message)
Takahashi, M.; Miyoshi, H.; Verma, I.M.; Gage, F.H., "Rescue from photoreceptor degeneration in the rd mouse by human immunodeficiency virus vector-mediated gene transfer," J. Virol. 73: 7812-7816 (Sept. 1999)
Uchida, N., Sutton, R.E., Friera, A.M., He, D., Reitsma, M.J., Chang, W.C., Veres, G., Scollay, R. & Weissman, I.L., "HIV, but not murine leukemia virus, vectors mediate high efficiency gene transfer into freshly isolated G0/G1 human hematopoietic stem cells," Proc. Natl Acad. Sci. USA. 95, 11939-11944 (1998)
Valentin, A., W. Lu, M. Rosati, R. Schneider, J. Albert, A. Karlsson and G. N. Pavlakis. "Dual effect of interleukin 4 on HIV-1 expression: Implications for viral phenotypic switch and disease progression," Proc. Natl Acad. Sci. USA. 95: 8886-91 (1998)
White, S.M., Renda, M, Nam, N-Y, Klimatcheva, E., Hu, Y, Fisk, J, Halterman, M, Rimel, B.J., Federoff, H, Pandya, S., Rosenblatt, J.D. and Planelles, V, "Lentivirus vectors using human and simian immunodeficiency virus elements," J. Virol. 73:2832-2840 (Apr. 1999)
Wolff, J.A. and Trubetskoy, V.S., "The Cambrian period of nonviral gene delivery," Nature Biotechnology 16:421-422 (1998)
Zolotukhin, J., Valentin, A., Pavlakis, G. N. and Felber, B. K. "Continuous propagation of RRE(-)and Rev(-)RRE(-) human immunodeficiency virus type 1 molecular clones containing a cis-acting element of Simian retrovirus type 1 in human peripheral blood lymphocytes," J. Virol. 68:7944-7952 (1994)
Zufferey, R., Nagy, D., Mandel, R.J., Naldini, L. and Trono, D., "Multiply Attenuated Lentiviral Vector Achieves Efficient Gene-Delivery In Vivo", Nature Biotechnology 15:871-875 (1997)
Zufferey, R., Dull, T., Mandel, R.J., Bukovsky, A., Quiroz, D., Naldini, L. & Trono, D., "Self-inactivating lentivirus vector for safe and efficient in vivo gene delivery," J. Virol. 72:9873-9880 (1998)

Those skilled in the art will recognize that any gene encoding a mRNA containing an inhibitory/instability sequence or sequences can be modified in accordance with the exemplified methods of this invention or their functional equivalents.

Modifications of the above described modes for carrying out the invention that are obvious to those of skill in the fields of genetic engineering, virology, immunology, medicine, and related fields are intended to be within the scope of the following claims.

Every reference cited hereinbefore throughout the application is hereby incorporated by reference in its entirety.

## Claims

1. A lentiviral expression system which is capable of functioning in the absence of Rev, Tat, and any viral RNA transport element comprising the following:
(a) a packaging vector comprising a HIV-1 *gag*/*pol* gene which has been mutated to eliminate inhibitory/instability regions;
(b) a transfer vector; and
(c) an envelope encoding vector.

2. A lentiviral expression system of Claim 1, wherein the HIV-1 *gag*/*pol* gene has the nucleotide sequence of SEQ ID NO:1.

3. A lentiviral expression system of Claim 1 or 2, wherein the packaging vector comprises a CMV promoter that controls expression of the *gag*/*pol* gene.

4. A transformed host cell comprising the lentiviral expression system of Claim 1,2 or 3.

5. A transformed host cell of Claim 4 wherein said cell is a eukaryote.

6. The host cell of Claim 5 wherein said cell is a human cell.

7. A process for making a lentiviral particle in the absence of Rev, Tat, or any viral RNA transport element comprising expressing HIV Gag and HIV Pol in a host cell from a HIV-1 *gag*/*pol* gene which has been mutated to eliminate inhibitory/instability regions and expressing an envelope protein from an envelope encoding gene whose expression is independent of Rev, Tat, or any viral RNA transport element.

8. A process for making a lentiviral particle comprising expressing HIV Gag and HIV Pol in a host cell from a vector comprising the nucleotide sequences encoding HIV Gag and HIV Pol corresponding to Seq. ID No. 1 in the presence of a gene encoding an envelope protein.

9. A lentiviral expression system that is Rev and Tat independent comprising the following:
(a) a packaging vector comprising an HIV *gag*/*pol* gene, or HIV *gag* gene and HIV *pol* gene, that has been mutated to eliminate inhibitory/instability regions, wherein the packaging vector, is capable of producing HIV Gag and Pol proteins in the absence of any RNA Transporter Elements;
(b) a transfer vector; and
(c) an envelope encoding vector.

10. A lentiviral expression system of claim 9, wherein the packaging vector comprises a CMV promoter that controls expression of the *gag*/*pol,* or *gag* and *pol,* genes.

## Patentansprüche

1. Lentivirales Expressionssystem, welches zur Erfüllung seiner Funktion in der Abwesenheit von Rev, Tat und irgendeines viralen RNA-Transportelements fähig ist, umfassend das Folgende:
(a) einen Verpackungsvektor, umfassend ein HIV-1-*gag*/*pol*-Gen, welches mutiert worden ist, um inhibitorische/Instabilitäts-Regionen zu beseitigen;
(b) einen Transfervektor; und
(c) einen für die Hülle codierenden Vektor.

2. Lentivirales Expressionssystem nach Anspruch 1, worin das HIV-1-*gag*/*pol*-Gen die Nukleotidsequenz der SEQ ID NR. 1 aufweist.

3. Lentivirales Expressionssystem nach Anspruch 1 oder 2, worin der Verpackungsvektor einen CMV-Promotor umfasst, der die Expression des *gag*/*pol*-Gens kontrolliert.

4. Transformierte Wirtszelle, umfassend das lentivirale Expressionssystem nach Anspruch 1, 2 oder 3.

5. Transformierte Wirtszelle nach Anspruch 4, wobei die Zelle eine Eukaryote ist.

6. Wirtszelle nach Anspruch 5, wobei die Zelle eine Humanzelle ist.

7. Verfahren zum Herstellen eines lentiviralen Partikels in der Abwesenheit von Rev, Tat oder irgendeines viralen RNA-Transportelements, umfassend das Exprimieren von HIV-Gag und HIV-Pol in einer Wirtszelle von einem HIV-1-*gag*/*pol-*Gen, welches mutiert worden ist, um inhibitorische/lnstabilitäts-Regionen zu beseitigen und ein Hüllprotein von einem für die Hülle codierenden Gen zu exprimieren, dessen Expression unabhängig von Rev, Tat oder irgendeinem viralen RNA-Transportelement ist.

8. Verfahren zum Herstellen eines lentiviralen Partikels, umfassend das Exprimieren von HIV-Gag und HIV-Pol in einer Wirtszelle von einem Vektor, umfassend die für HIV-Gag und HIV-Pol codierenden Nukleotidsequenzen entsprechend der SEQ ID Nr. 1, in der Gegenwart eines Gens, das für ein Hüllprotein codiert.

9. Lentivirales Expressionssystem, das Rev- und Tat-unabhängig ist, umfassend das Folgende:
(a) einen Verpackungsvektor, umfassend ein HIV-1-*gag*/*pol*-Gen, oder ein HIV-*gag*-Gen und ein HIV-*pol*-Gen, welches mutiert worden ist, um inhibitorische/lnstabilitäts-Regionen zu beseitigen, wobei der Verpackungsvektor zum Produzieren der HIV-Gag- und -Pol-Proteine in der Abwesenheit irgendeines RNA-Transporterelements fähig ist;
(b) einen Transfervektor; und
(c) einen für die Hülle codierenden Vektor.

10. Lentivirales Expressionssystem nach Anspruch 9, wobei der Verpackungsvektor einen CMV-Promotor umfasst, der die Expression des *gag*/*pol*-Gens, oder der *gag*- und *pol*-Gene, kontrolliert.

## Revendications

1. Système d'expression lentivirale qui est apte à fonctionner en l'absence de Rev, Tat, et de tout élément de transport d'ARN viral, comprenant les suivants :
(a) un vecteur d'encapsidation comprenant un gène *gag*/*pol* du VIH-1 qui a été muté pour éliminer les régions inhibitrices/instables ;
(b) un vecteur de transfert ; et
(c) un vecteur codant l'enveloppe.

2. Système d'expression lentivirale selon la revendication 1, dans lequel le gène *gag*/*pol* du VIH-1 a la séquence nucléotidique de la SEQ ID NO: 1.

3. Système d'expression lentivirale selon la revendication 1 ou 2, dans lequel le vecteur d'encapsidation comprend un promoteur de CMV qui contrôle l'expression du gène *gag*/*pol.*

4. Cellule hôte transformée comprenant le système d'expression lentivirale selon la revendication 1, 2 ou 3.

5. Cellule hôte transformée selon la revendication 4, ladite cellule étant un eucaryote.

6. Cellule hôte selon la revendication 5, ladite cellule étant une cellule humaine.

7. Procédé pour préparer une particule lentivirale en l'absence de Rev, Tat ou de tout élément de transport d'ARN viral, comprenant l'expression de Gag du VIH et de Pol du VIH dans une cellule hôte par un gène *gag*/*pol* du VIH-1 qui a été muté pour éliminer les régions inhibitrices/instables et l'expression d'une protéine d'enveloppe par un gène codant l'enveloppe dont l'expression est indépendante de Rev, Tat ou de tout élément de transport d'ARN viral.

8. Procédé pour préparer une particule lentivirale, comprenant l'expression de Gag du VIH ou Pol du VIH dans une cellule hôte par un vecteur comprenant les séquences nucléotidiques codant Gag du VIH et Pol du VIH correspondant à la Séq. ID No. 1 en présence d'un gène codant une protéine d'enveloppe.

9. Système d'expression lentivirale qui est indépendant de Rev et Tat, comprenant les suivants :
(a) un vecteur d'encapsidation comprenant un gène *gag*/*pol* du VIH-1 ou un gène *gag* du VIH et un gène *pol* du VIH, qui a été muté pour éliminer les régions inhibitrices/instables, le vecteur d'encapsidation étant apte à produit les protéines Gag et Pol du VIH en l'absence de tous éléments transporteurs d'ARN ;
(b) un vecteur de transfert ; et
(c) un vecteur codant l'enveloppe.

10. Système d'expression lentivirale selon la revendication 9, dans lequel le vecteur d'encapsidation comprend un promoteur de CMV qui contrôle l'expression des gènes *gag*/*pol,* ou *gag* et *pol.*
